# EUROPEAN PATENT SPECIFICATION

(11) **EP 1 380 642 B1**
(45) Date of publication and mention of the grant of the patent: **31.03.2010**
(21) Application number: 03008719.1
(22) Date of filing: 16.04.2003
(51) Int. Cl.: C12N 15/10, B01L 3/00

(54) **A method for the separation and purification of nucleic acid**
Methode zur Trennung und Aufreinigung von Nukleinsäuren
Méthode de séparation et purification des acides nucléiques

(30) Priority: 10.07.2002 JP 2002201106
(43) Date of publication of application: 14.01.2004
(73) Proprietor: FUJIFILM Corporation, Minato-ku Tokyo (JP)
(72) Inventor: Mori, Toshihiro, Asaki-shi, Saitama 351-8585 (JP); Takeshita, Yumiko, Asaki-shi, Saitama 351-8585 (JP); Makino, Yoshihiko, Asaki-shi, Saitama 351-8585 (JP)
(74) Representative: Hiebl, Inge Elisabeth

(56) References cited:
- EP-A- 0 630 974
- EP-A- 1 316 606
- CH-A- 653 043
- US-A- 4 098 931
- US-A- 5 019 347
- US-A- 5 804 684
- SCHMALZING D ET AL: "CHARACTERIZATION AND PERFORMANCE OF A NEUTRAL HYDROPHILIC COATING FOR THE CAPILLARY ELECTROPHORETIC SEPARATION OF BIOPOLYMERS" JOURNAL OF CHROMATOGRAPHY A, ELSEVIER SCIENCE, NL, vol. 652, no. 1, 1993, pages 149-159, XP009005046 ISSN: 0021-9673
- THE COLLABORATIVE GROUP, LTD.: CELLUFLOW TA-25 & C-25, [Online] - 7 June 2002 (2002-06-07) XP002260589 Retrieved from the Internet: <URL:http://web.archive.org/web/2002101513 3217/http://www.collabo.com/celluflow.htm> [retrieved on 2003-11-07]
- MAKING RAYON FIBER, [Online] 1999, XP002251959 Retrieved from the Internet: <URL:http://www.mindfully.org/Plastic/Cell ulose/Rayon-Fiber.htm> [retrieved on 2003-08-21]

### SEQUENCE LISTING

<110> Fuji Photo Film Co., Ltd.
<120> A method for the separation and purification of nucleic acid
<130> 13341EP
<140> EP 03 008 719.1 <141> 2003-04-16
<150> JP 201106/2002 <151> 2002-07-10
<160> 10
<170> PatentIn Ver. 2.1
<210> 1
   <211> 20
   <212> DNA
   <213> Artificial Sequence
<220>
   <223> Description of Artificial Sequence:Primer
<400> 1
   gatccgtcat aaagtcaaac 20
<210> 2
   <211> 20
   <212> DNA
   <213> Artificial Sequence
<220>
   <223> Description of Artificial Sequence:Primer
<400> 2
   ggatgagaat ggaatcctat 20
<210> 3
   <211> 29
   <212> DNA
   <213> Artificial Sequence
<220>
   <223> Description of Artificial Sequence:Primer
<400> 3
   cacctgcaga tgtgggtggc accctgcca 29
<210> 4
   <211> 30
   <212> DNA
   <213> Artificial Sequence
<220>
   <223> Description of Artificial Sequence:Primer
<400> 4
   gtggaattca ctcgccactg cctgggtctc 30
<210> 5
   <211> 21
   <212> DNA
   <213> Artificial Sequence
<220>
   <223> Description of Artificial Sequence:Primer
<400> 5
   gtggctttcc tgaagctgtt c 21
<210> 6
   <211> 21
   < 212> DNA
   <213> Artificial Sequence
<220>
   <223> Description of Artificial Sequence:Primer
<400> 6
   gatgccgttg gcctggtcga c 21
<210> 7
   <211> 27
   <212> DNA
   <213> Artificial Sequence
<220>
   <223> Description of Artificial Sequence:Primer
<400> 7
   gcgatgctgt aatgactctc gacaagc 27
<210> 8
   <211> 25
   <212> DNA
   <213> Artificial Sequence
<220>
   <223> Description of Artificial Sequence:Primer
<400> 8
   ggtctgcaaa ttctgcggcg tcgtc 25
<210> 9
   <211> 20
   <212> DNA
   <213> Artificial Sequence
<220>
   <223> Description of Artificial Sequence:Primer
<400> 9
   gcgctgctca gatagcgatg 20
<210> 10
   <211> 19
   <212> DNA
   <213> Artificial Sequence
<220>
   <223> Description of Artificial Sequence:Primer
<400> 10
   ggagggccac tgacaacca 19

## Description

### TECHNICAL FIELD

The present invention relates to a method for isolating and purifying nucleic acids, a unit for isolation and purification of nucleic acid, and an analytical method using the same.

### BACKGROUND ART

Various forms of nucleic acids are used in a variety of fields. For example, in the field of recombinant nucleic acid technology, nucleic acids are used in the form of probes, genomic nucleic acids and plasmid nucleic acids.

In the field of diagnostics, nucleic acids are used in various methods. For example, nucleic acid probes are used routinely in the detection and diagnosis of human pathogen. Likewise, nucleic acids are used in the detection of genetic disorders. Nucleic acids are also used in the detection of food contaminants. Further, nucleic acids are used routinely in locating, identifying and isolating nucleic acids of interest for a variety of reasons ranging from genetic mapping to cloning and recombinant expression.

In many cases, nucleic acids are available in extremely small amounts, and thus isolation and purification procedures are laborious and time consuming. These often time consuming and laborious operations are likely to lead to the loss of nucleic acids. In purifying nucleic acids from samples obtained from serum, urine and bacterial cultures, there is a risk of contamination and false positive results.

One widely known purification method is a method of adsorbing nucleic acids onto surfaces of silicon dioxide, silica polymers, magnesium silicate and the like, followed by the procedures such as washing and desorbing to carry out purification (e.g., JP Patent Publication (Examined Application) No. 7-51065). This method exhibits excellent isolation ability, but industrial mass production of adsorbents with identical performance is difficult. Further, there are other drawbacks, such as inconvenience in handling and difficulty in processing into various shapes.

US 5,804,684 discloses a method of isolating nucleic acid in a substantially purified form, including the steps of: a) contacting a biological sample which contains aggregated nucleic acid with a matrix comprising a solid hydrophilic organic polymer without an effective positive charge under conditions which permit the nucleic acid to bind to the matrix; and b) recovering nucleic acid from the matrix.

EP 0 630 974 discloses a method for the detection of a target nucleic acid comprising:
A) amplifying a target nucleic acid in a reaction mixture containing primers specific to and hybridisable with the opposing strands of a target nucleic acid, a DNA polymerase and one or more dNTP's so as to generate inorganic orthophosphate, and B) detecting the presence of inorganic orthophosphate generated in said reaction mixture from the amplification by contacting the inorganic orthophosphate with a reagent which provides a colorimetric signal in response to inorganic orthophosphate, as an indication of the presence of the target nucleic acid.

EP 1316 606 discloses a method for separation and purification of nucleic acid comprising the steps of adsorbing nucleic acid onto a solid phase and desorbing the nucleic acid from the solid phase, wherein the solid phase comprises a base material in which a graft polymer chain having a hydrophilic group is bonded onto the surface.

### DISCLOSURE OF THE INVENTION

It is an object of the present invention to provide a method for isolating and purifying nucleic acids, which comprises adsorbing nucleic acids in a sample onto a solid phase surface and desorbing the nucleic acids by washing and the like. It is another object of the present invention to provide: a method for isolating and purifying nucleic acids which employs a solid phase wherein the solid phase has excellent isolating capability, good washing efficiency, and easy workability, and can be mass produced with substantially identical isolating capability; and a unit for isolation and purification of nucleic acid which is suitable for carrying out said methods It is another object of the present invention to provide a method for analyzing nucleic acid which employs the above method for isolating and purifying nucleic acids. It is yet another object of the present invention to provide a method for analyzing nucleic acid fragment
wherein the method can be conducted conveniently and expeditiously using a small apparatus without the need for special techniques, complicated operations and specific apparatus, that is, a method for analyzing nucleic acid fragment which can be automated and requires as little space as possible.

The present inventors have made intensive studies to solve the above problems.
As a result, they have found that, in a method for isolating and purifying nucleic acids comprising adsorbing and desorbing nucleic acids onto and from a solid phase, nucleic acids can be isolated with a high purity from a sample solution containing nucleic acids, by using as the solid phase a porous surface-saponified cellulose triacetate membrane having a surface saponification ratio of 50% or 100%, which is present in one piece and using a unit for isolation and purification of nucleic acid which comprises a container having two openings and containing the solid phase. Further, they have found that nucleic acid analysis with excellent convenience and prompt efficiency can be conducted without the need for any specific apparatus by detecting, by the use of dry analytical element, pyrophosphoric acid produced during polymerase elongation reaction with the use of the nucleic acid isolated in the above method. The present invention has been accomplished based on these findings.

Thus, according to the present invention, there is provided a method for isolating and purifying a nucleic acid, comprising the steps of(a) adsorbing a nucleic acid onto a solid phase being a porous surface-saponified cellulose triacetate membrane having a surface saponification ratio of 50% or 100%, which is present in one piece and (b) desorbing the nucleic acid from the solid phase.

According to another aspect of the present invention, there is provided a unit for isolation and purification of nucleic acid comprising a container which has at least two openings and contains a solid phase being a porous surface-saponified cellulose triacetate membrane having a surface saponification ratio of 50% or 100%, which is present in one piece.

Further disclosed is a method for introducing a hydroxyl group into acetylcellulose wherein beads are coated with acetylcellulose and then a surface thereof is saponified.

Further disclosed are beads having on a surface thereof acetylcellulose membrane into which hydroxyl group is introduced by surface saponification.

According to another aspect of the present invention, there is provided a method for analyzing nucleic acid which comprises the steps of:
(1) isolating and purifying a nucleic acid fragment containing a target nucleic acid fragment by the above-mentioned method of the present invention;
(2) allowing the target nucleic acid fragment, at least one primer complementary to a portion of the target nucleic acid fragment, at least one deoxynucleoside triphosphate, and at least one polymerase to react with each other, to conduct polymerase elongation reaction with using the target nucleic acid fragment as a template and using 3' terminal of the primer as initiation site; and
(3) detecting whether polymerase elongation reaction proceeds or whether the polymerase elongation reaction product hybridizes with another nucleic acid.

According to another aspect of the present invention, there is provided an analytical apparatus for conducting the method for analyzing nucleic acid of the present invention, which comprises: (1) means for extracting and purifying nucleic acid, which comprises a unit for isolation and purification of nucleic acid of the present invention; (2) reaction means for conducting polymerase elongation reaction; and (3) means for detecting whether polymerase elongation reaction proceeds or whether the polymerase elongation reaction product hybridizes with another nucleic acid.

### BRIEF DESCRIPTION OF THE DRAWINGS

Fig. 1 is a schematic view of a unit for isolation and purification of nucleic acid of the present invention.
Fig. 2 is one example of a unit for isolation and purification of nucleic acid of the present invention. A differential pressure generator to be connected to an opening 21 is not shown in the figure. Fig. 2 shows a container 1, a main body 10, an opening 101, a bottom face 102, a frame 103, a wall 104, a step 105, spaces 121, 122, and 123, a depressing member 13, a hole 131, projections 132, a lid 20, an opening 21 and a solid phase 30.
Fig. 3 is a schematic view illustrating the analysis of nucleic acid according to the present invention.
Fig. 4 is a perspective view illustrating an example of a kit in the form of cartridge which can be used in the present invention. Fig. 4 shows a kit 10, a base body 21, a lid 22, an opening 31, a reaction cell 32, a detection unit 33, canaliculus 41 and 42, a dry analytical element 51, a primer 81, deoxynucleoside triphosphate (dNTP) 82, and polymerase 83.
Fig- 5 is a perspective view illustrating a system configuration when using a kit, in the form of cartridge, of the present invention. Fig. 5 shows the kit 10, the reaction cell 32, the detection unit 33, temperature control portions 61 and 62, and detection units 71 and 72.
Fig. 6 shows the results of purification of nucleic acid from whole blood sample using a porous cellulose triacetate membrane having 100% surface saponification.
Fig. 7 shows the results of electrophoresis of PCR products using nucleic acids isolated and purified in accordance with the method of the present invention.
Fig. 8 illustrates the relationship between the number of added *Pseudomonas syringae* and the optical density of reflecting light (OD_{R})
Fig. 9 illustrates the relationship between the number of added *Pseudomonas syringae* and the optical density of reflecting light (OD_{R}).

### DETAILED DESCRIPTION OF THE INVENTION

Hereinafter, embodiments of the present invention will be described.

### (1) Method for isolating and purifying nucleic acid according to the present invention

A method for isolating and purifying nucleic acid according to the present invention is characterized in that the method comprises adsorbing and desorbing nucleic acids onto and from a solid phase being a surface-saponified cellulose triacetate membrane having a surface saponification ratio of 50% or 100%, which is present in one piece.

"Nucleic acid" in the present invention may be single stranded or double stranded, and there is no limit as to the molecular weight thereof.

The expression "surface-saponification" used herein means that only the surface which comes in contact with saponification treatment solution (e.g., NaOH) is saponified. In the present invention, it is preferable that only a surface of a solid phase is saponified while the structure of the solid phase remains cellulose acetate. This allows the amount of hydroxyl group (density) on the surface of the solid phase to be controlled according to the degree of surface saponification treatment (surface saponification degree).

With the porous membrane, the structure of the membrane remains cellulose acetate, and only a surface of the structure is preferably saponified. This allows the spatial amount (density) of hydroxyl groups to be controlled according to the degree of surface-saponification treatment (surface saponification degree) x pore size. Further, the structure of the membrane is composed of cellulose acetate and therefore a rigid solid phase can be obtained. Here, the introduction of hydroxyl groups on only a surface by surface-saponification of cellulose acetate means that the structure remains cellulose acetate and the surface is made into cellulose. It is noted that when cellulose is used as a raw material, a porous membrane or a flat membrane cannot industrially be produced because cellulose cannot be made into a liquid state.

For example, cellulose triacetate membrane is commercially available from Fuji Photo Film Co., Ltd. under the tradename of "TAC base." As a porous cellulose triacetate membrane, MICROFILTER FM500 (Fuji Photo Film Co., Ltd.) is available.

In addition, it is preferable that, for example, cellulose triacetate membranes are formed on surfaces of polyethylene beads and the resultant beads are surface-saponified so as to have a hydroxyl group on the surface. In this case, the beads are coated with cellulose triacetate. Any material may be used as beads as long as it does not contaminate nucleic acids, and it is not limited to polyethylene.

In order to increase isolation efficiency of nucleic acids, it is preferable that a larger number of hydroxyl groups are present. For example, in the case of cellulose acetate such as cellulose triacetate, the surface-saponification ratio is preferably 5 % or more, more preferably 10% or more.

For surface-saponifying cellulose acetate, the object to be surface-saponified is soaked in an aqueous solution of sodium hydroxide. The surface-saponification ratio may be changed by changing the concentration of sodium hydroxide. The surface-saponification ratio is determined by quantifying residual acetyl groups by means of NMR.

In the method for isolating and purifying nucleic acids according to the present invention, it is preferred that nucleic acids are adsorbed and desorbed by using a unit for isolation and purification of nucleic acid which comprises a container having at least two openings wherein the container contains a solid phase being a porous surface-saponified cellulose triacetate membrane having a surface saponification ratio of 50% or 100%, which is present in one piece.

Further preferably, nucleic acids can be adsorbed and desorbed by using a unit for isolation and purification of nucleic acid which comprises: (a) a solid phase being a porous surface-saponified cellulose triacetate membrane having a surface saponification ratio of 50% or 100%, which is present in one piece; (b) a container having at least two openings and containing the solid phase; and (c) a differential pressure generator connected to one opening of the container.

According to a first embodiment of the present invention, a method for isolating and purifying nucleic acids comprises the following steps of
:(a) preparing a sample solution containing nucleic acids by use of a sample inserting one opening of a unit for isolation and purification of nucleic acid into a sample solution containing nucleic acids;
(b) creating a reduced pressure condition in a container by a differential pressure generator connected to another opening of the unit for isolation and purification of nucleic acid, sucking the sample solution containing nucleic acids, and allowing the sample solution to come into contact with a solid phase being a porous surface-saponined cellulose triacetate membrane having a surface saponification ratio of 50% or 100%, which is present in one piece;
(c) creating an increased pressure condition in the container by the differential pressure generator connected to said another opening of the unit for isolation and purification of nucleic acid, and discharging the sucked sample solution containing nucleic acids out of the container;
(d) inserting said one opening of the unit for isolation and purification of nucleic acid into a nucleic acid washing buffer solution;
(e) creating a reduced pressure condition in the container by the differential pressure generator connected to said another opening of the unit for isolation and purification of nucleic acid, sucking the nucleic acid washing buffer solution, and allowing the buffer solution to come into contact with the solid phase.
(f) creating an increased pressure condition in the container by the differential pressure generator connected to said another opening of the unit for isolation and purification of nucleic acid, and discharging the sucked nucleic acid washing buffer solution out of the container;
(g) inserting the one opening of the unit for isolation and purification of nucleic acid into a solution capable of desorbing nucleic acids from the solid phase;
(h) creating a reduced pressure condition in the container by the differential pressure generator connected to said another opening of the unit for isolation and purification of nucleic acid, sucking the solution capable of desorbing nucleic acids from the solid phase;
   , and allowing the solution to come into contact with the solid phase; and
(i) creating a increased pressure condition in the container by the differential pressure generator connected to said another opening of the unit for isolation and purification of nucleic acid, and discharging out of the container the solution capable of desorbing nucleic acids from the solid phase.

According to a second embodiment of the present invention, a method for isolating and purifying nucleic acids comprises the following steps of:
(a) preparing a sample solution containing nucleic acids from a sample, and injecting the sample solution containing nucleic acids in one opening of a unit for isolation and purification of nucleic acid;
(b) creating a increased pressure condition in the container by a differential pressure generator connected to said one opening of the unit for isolation and purification of nucleic acid, discharging the injected sample solution containing nucleic acids out of another opening, and thereby allowing the sample solution to come into contact with a solid phase being a porous surface-saponified cellulose triacetate membrane having a surface saponification ratio of 50% or 100%, which is present in one piece;
(c) injecting a nucleic acid washing buffer in said one opening of the unit for isolation and purification of nucleic acid;
(d) creating an increase pressure condition in the container by the differential pressure generator connected to said one opening of the unit for isolation and purification of nucleic acid, discharging the injected nucleic acid washing buffer out of said another opening, and thereby allowing the buffer to come into contact with the solid phase;
(e) injecting a solution capable of desorbing nucleic acids from the solid phase in the one opening of the unit for isolation and purification of nucleic acid;
(f) creating an increased pressure condition in the container by the differential pressure generator connected to said one opening of the unit for isolation and purification of nucleic acid, discharging the injected solution capable of desorbing nucleic acids out of said another opening, and thereby desorbing nucleic acids from the solid phase and discharging the desorbed nucleic acids out of the container.

Detailed description will be provided concerning a method for isolating and purifying nucleic acids using a porous surface-saponified cellulose triacetate membrane having a surface saponification ratio of 50% or 100%, which is present in one piece. According to the present invention, preferably a sample solution containing nucleic acids is allowed to come into contact with a solid phase being a porous surface-saponified cellulose triacetate membrane having a surface saponification ratio of
50% or 100%, which is present in one piece, and nucleic acids in the sample solution are adsorbed onto the solid phase. Next, the nucleic acids adsorbed onto the solid phase are desorbed therefrom using a suitable solution described below. More preferably, the sample solution containing nucleic acids is a solution which is prepared by treating a sample containing cells or viruses with a solution capable of dissolving cell membranes and nuclear membranes so as to disperse nucleic acids into the solution, and then adding a water-soluble organic solvent to the solution.

There is no limit on the sample solutions containing nucleic acids which can be used in the present invention, but examples thereof in the field of diagnostics include body fluids collected as samples, such as whole blood, plasma, serum, urine, stool, sperm, and saliva, or plants (or a part thereof), animals (or a part thereof), solutions prepared from biological materials such as lysates and homogenates of the above samples.

First, these samples are treated with an aqueous solution comprising a reagent which dissolves cell membranes and solubilizes nucleic acids. This enables cell membranes and nuclear membranes to be dissolved, and enables nucleic acids to be dispersed into the aqueous solution.

For dissolving cell membranes and solubilizing nucleic acids, for example, when a sample is whole blood, (1) removal of erythrocytes, (2) removal of various proteins, and (3) lysis of leukocytes and nuclear membrane, are necessary. (1) Removal of erythrocytes and (2) removal of various proteins are necessary to prevent their non-specific adsorption onto the solid phase and clogging of a porous membrane, and (3) lysis of leukocytes and nuclear membranes is necessary to solubilize nucleic acids which are to be extracted. In particular, (3) lysis of leukocytes and nuclear membranes is an important process, and in the method of the present invention, nucleic acids are required to be solubilized in this process. In Examples described hereinafter, guanidine hydrochloride, Triton-X100, and protease K (SIGMA) are added, and then the mixture is incubated at 60°C for 10 minutes, thereby accomplishing the above (1), (2), and (3) simultaneously.

As the reagent to be used in the present invention for solubilizing nucleic acids, a solution comprising a guanidine salt, a surfactant and a protease may be used.

Guanidine hydrochloride is preferable as the guanidine salt, but other guanidine salts (e.g., guanidine isothiocyanate and guanidine thiocyanate) can be used. The concentration of guanidine salt in the solution is from 0.5 M to 6 M, preferably from 1M to 5 M.

As the surfactant, Triton-X100 can be used, and also usable are anionic surfactants such as SDS, sodium cholate and sodium sarcosinate, nonionic surfactants such as Tween 20 and MEGAFAC, and other various amphoteric surfactants. In the present invention, nonionic surfactants such as polyoxyethylene octyl phenyl ether (Triton-X100) are preferably used. The concentration of the surfactant in the solution is usually 0.05 % by weight to 10 % by weight, more preferably 0:1 % by weight to 5 % by weight.

Although protean K can be used as a protease, other proteases can also produce the same effect. Proteases are preferably heated because they are enzymes, and therefore they are used preferably at 37°C to 70°C, more preferably 50°C to 65°C.

A water-soluble organic solvent is added to the solution wherein nucleic acids are dispersed as described, thereby enabling contact with the porous surface-saponified cellulose triacetate membrane having a surface saponification ratio of 50% or 100%, which is present in one piece. This operation enables nucleic acids in the sample solution to be adsorbed onto said membrane. In order to adsorb the solubilized nucleic acids onto the solid phase being a porous surface-saponified cellulose triacetate membrane having a surface saponification ratio of 50% or 100%, which is present in one piece in the operations described above, it is necessary to mix a water-soluble organic solvent with a mixed solution of solubilized nucleic acids and to allow salts to be present in the obtained mixed solution of nucleic acids.

Namely, nucleic acids are solubilized under unstable conditions by breaking hydration structure of water molecules existing around nucleic acids. When nucleic acids under this condition is made to come into contact with the above solid phase, it is considered that polar groups on the surface of nucleic acids interact with polar groups of the solid phase surface, and nucleic acids are adsorbed onto the solid phase surface. In the method of the present invention, nucleic acids can be placed under unstable condition by mixing a water-soluble organic solvent with the mixed solution of solubilized nucleic acids and by allowing salts to be present in the obtained mixture solution of nucleic acids.

Examples of the water-soluble organic solvent to be used herein include ethanol, isopropanol, and propanol. Among these, ethanol is preferable. The concentration of the water-soluble organic solvent is preferably 5% by weight to 90 % by weight, more preferably 20% by weight to 60% by weight. It is particularly preferable that ethanol is added so as to have as a high concentration as possible, but to such extent that aggregation does not occur.

As the salts existing in the obtained mixture solution of nucleic acids, preferable are various chaotropic substances (guanidine salts, sodium iodide, and sodium perchlorate), sodium chloride, potassium chloride, ammonium chloride, sodium bromide, potassium bromide, calcium bromide, ammonium bromide and the like. Guanidine salts are in particular preferable because they exhibit effects for both the dissolution of cell membranes and the solubilization of nuclear acids.

Next, the porous surface-saponified cellulose triacetate membrane having a surface saponification ratio of 50% or 100%, which is present in one piece, onto which nucleic acids are adsorbed, is made to come into contact with a nucleic acid washing buffer solution. This solution washes out impurities which were present in the sample solution and were adsorbed onto the organic high polymer having a hydroxyl group on a surface thereof along with nucleic acids. Therefore, the buffer solution is required to have such a composition that only impurities and not nucleic acids are desorbed from the organic high polymer having a hydroxyl group on a surface thereof.
The nucleic acid washing buffer solution comprises a solution which contains a base agent and a buffer agent, and if further necessary, a surfactant. Examples of the base agent include aqueous solutions having approximately 10 to 100 % by weight of methanol, ethanol, isopropanol, n-isopropanol, butanol, actone, or the like (preferably approximately 20 to 100 % by weight, more preferably approximately 40 to 80 % by weight). Examples of the buffer agents and surfactants include those described above. Among these, a solution comprising ethanol, Tris, and Triton-X100 is preferable. Tris and Triton-X100 preferably have a concentration of 10 to 100 mM and 0.1 to 10 % by weight, respectively.

Further, the washed porous surface-saponified cellulose triacetate membrane having a surface saponification ratio of 50% or 100%, which is present in one piece, is made to come into contact with a solution capable of desorbing nucleic acids which have been adsorbed onto the above membrane. The solution is collected since it contains nucleic acids of interest, and is then used for the ensuing operations such as PCR (polymerase chain reaction) to amplify nucleic acids. The solution capable of desorbing nucleic acids has preferably a low salt concentration, and more preferably the solution having a salt concentration of 0.5 M or lower is used. Examples of the solution to be used include purified distilled water and TE buffer.

### (2) Unit for isolation and purification of nucleic acid according to the present invention

A unit for isolation and purification of nucleic acid of the present invention comprises a container having at least two openings wherein the container contains a solid phase being a porous surface-saponified cellulose triacetate membrane having a surface saponification ratio of 50% or 100%, which is present in one piece.

A material for the container is not limited, as long as the container can contain the above solid phase and have two or more openings provided thereon. Plastics are preferable due to their easy production. For example, preferably used are transparent or opaque resins such as polystyrene, polymethacrylic acid ester, polyethylene, polypropylene, polyester, nylon, and polycarbonate.

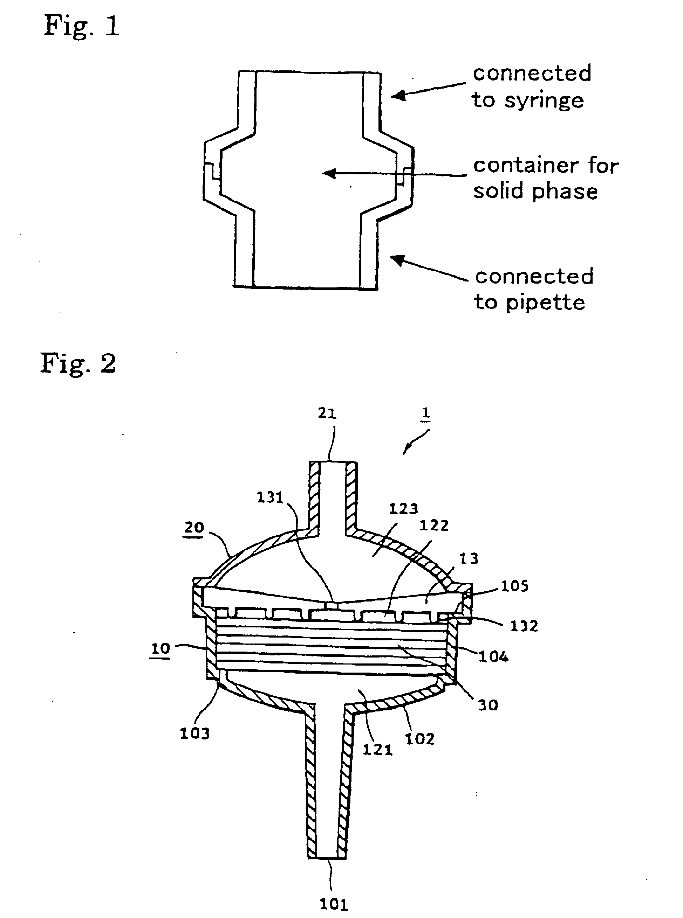

Fig. 1 shows a schematic view of the container. Basically, the container comprises a portion capable of containing the solid phase, and the solid phase is maintained inside the containing portion while sucking and discharging a sample solution and the like. Further, the opening of the container may be connectable to a differential pressure generator such as an injector. To this end, it is preferable that the container originally comprises two separated parts, and after the solid phase is containd the separated parts are incorporated with each other. Moreover, in order to prevent the solid phase from coming out of the containing portion, meshes prepared by materials which do not contaminate DNA may be placed on top of and beneath the solid phase.

There is no specific limit on the shape of the porous surface-saponified cellulose triacetate membrane, which is contained in the container, and any shape may be employed such as round shape, square shape, rectangle shape, and oval shape, tubulous shape and scrolling shape in the case of membrane, or beads coated with surface-saponified cellulose triacetate. However, because of production suitability, a highly symmetric shape such as round, square, tubulous, or scrolling shape, and beads are preferable.

One opening of the container is inserted into the sample solution containing nucleic acids so as to suck the sample solution and to bring it into contact with the the porous surface-saponified cellulose triacetate membrane, and the sample solution is discharged. Next, the nucleic acid washing buffer solution is sucked and discharged. Then, the solution capable of desorbing nucleic acids from the porous surface-saponified cellulose triacetate membrane is sucked and discharged, and the discharged solution is collected to obtain nucleic acids of interest.

The nucleic acids of, interest may be obtained by soaking the porous surface-saponified cellulose triacetate membrane into the sample solution containing nucleic acids, the nucleic acid washing buffer solution, and the solution capable of desorbing nucleic acids from the porous surface-saponified cellulose triacetate membrane in this order

The unit for isolation and purification of nucleic acid of the present invention preferably comprises: (a) a solid phase being a porous surface-saponified cellulose triacetate membrane having a surface saponification ratio of 50% or 100%, which is present in one piece; (b) a container having at least two openings and containing the solid phase; and (c) a differential pressure generator connected to one opening of the container. Hereinafter, the unit for isolation and purification of nucleic acid will be described.

The container is usually prepared in the form of having a separate lid and a main body for containing a solid phase being a porous surface-saponified cellulose triacetate membrane having a surface saponification ratio of 50% or 100%, which is present in one piece, and at least one opening is provided for each of the main body and lid. One opening is used as an inlet and outlet for sample solutions containing nucleic acids, a nucleic acid washing buffer solution, and a solution capable of desorbing nucleic acids from the solid phase (hereinafter referred to as "sample solution and the like"), while the other opening is connected to a differential pressure generator which creates a reduced or increased pressure condition in the container. Although the shape of the main body is not particularly limited, it preferably has a round cross section in order to make the production easy and readily disperse the sample solution and the like over the entire face of the solid phase. It is also preferable that the container has a rectangle cross section in order to prevent cutting chips of the solid phase from being produced.

The lid is required to be joined with the main body so as to enable the inside of the container to have a reduced and increased pressure condition by a differential pressure generator. However, as long as this condition is provided, any joint method may be selected Examples of the methods include use of an adhesive, thread-in method, built-in method, screw cramp, and fusion bond by ultrasonic heating.

The internal volume of the container is determined solely by the amount of the sample solution to be treated. In general, it is represented by a volume of the solid phase to be containd therein. Namely, the container preferably has a size enough to contain approximately 1 to 6 pieces of solid phase which has a thickness of approximately 1 mm or less (e.g., approximately 50 to 500 µm) and a diameter of approximately 2 mm to 20 mm,

It is preferable that the end face of solid phase is closely contacted to an inner wall of the container to such extent that the sample solution and the like do not pass through the space between them.

There is provided a space from the inner wall of the container, rather than being closely contacted therewith, beneath the solid phase facing the opening used as inlet for the sample solution and the like, so that the container has such a structure that the sample solution and the like can be dispersed over the entire solid phase as evenly as possible.

There is preferably provided a member with a hole almost in its center on the solid phase facing the other opening, that is the opening connected to the differential pressure generator. This member depresses the solid phase and also effectively discharges the sample solution and the like. The member preferably has a shape with a slant, for example, like a funnel or a bowl in order to congregate liquid to its center.
The size of the hole, the degree of the slant, and the thickness of the member may be properly determined by those skilled in the art, considering the amount of sample solution to be treated or the size of container for containing the solid phase. There is preferably provided a space between this member and the other opening for storing the overflowed sample solution and the like in the space and preventing overflowed sample solution and the like from being sucked into the differential pressure generator. The size of this space is also appropriately determined by those skilled in the art. For efficient collection of nucleic acids, it is preferable to suck more amount of the sample solution containing nucleic acids than the amount needed for soaking the entire solid phase.

Moreover, it is preferable to provide a space between the solid phase and this member, in order to prevent the sample solution and the like from gathering only directly underneath the opening while sucking and to allow the sample solution and the like to pass through the inside of the solid phase in a relatively even manner. To this end, plural projections are preferably provided from the member toward the solid phase.
Although the size or number of the projections can be determined by those skilled in the art, it is preferable to maintain as large an open area of the solid phase as possible while maintaining the space.

When there are provided three or more openings on the container, it is needless to say that extra openings must be closed temporarily in order to enable liquid to be sucked and discharged by the reduction and increase of pressure.

The differential pressure generator first reduces the pressure inside the container containing the solid phase so as to suck the sample solution containing nucleic acids. Examples of the differential pressure generator include an injector, a pipette, and a pump such as Perista pump which can conduct suction and pressure increase. Among these, an injector is preferable for manual operation, and a pump is preferable automatic operation Further, a pipette is advantageous since it can easily be handled by one hand. Preferably, the differential pressure generator is detachably connected to one opening of the container.

Next, a method for purification of nucleic acid using the unit for isolation and purification of nucleic acid will be described. First, one opening of the unit for isolation and purification of nucleic acid is inserted into a sample solution containing nucleic acids. Then, the sample solution is sucked into the container by reducing the pressure inside the purification unit by means of the differential pressure generator connected to another opening. By this operation, the sample solution is made to come into contact with the solid phase so that nucleic acids present in the sample solution are adsorbed onto the solid phase. In this case, the amount of sample solution to be sucked is preferably enough to come into contact with almost the entire solid phase. However, suction of the sample solution into the differential pressure generator would cause contamination, and thus the amount is to be adjusted accordingly.

After sucking an appropriate amount of sample solution, the sucked solution is discharged by increasing the pressure inside the container of the unit by means of the differential pressure generator. It is not necessary to provide an interval untill this operation, and thus the solution may be discharged immediately after sucking.

Next, the nucleic acid washing buffer solution is sucked into the container and discharged therefrom so as to wash the inside the container by reducing and increasing the pressure in the same manner as described above. This solution can wash out residual sample solution inside the container, and at the same time it works to wash out impurities of the sample solution, which are adsorbed onto the solid phase along with nucleic acids. Therefore, it must have such a composition that it desorbs only impurities from the solid phase, and not the nucleic acids. The nucleic acid washing buffer solution comprises an aqueous solution which contains a base agent and a buffer, and if necessary, a surfactant. Examples of the base agent include aqueous solutions having approximately 10 to 90 % (preferably approximately 50 to 90 %) of methyl alcohol, ethyl alcohol, butyl alcohol, acetone, or the like. Examples of the buffer and surfactant include those described above. Among those, solutions containing ethyl alcohol, Tris and Triton-X100 are preferable. Preferable concentrations of Tris and Triton-X100 are 10 to 100 mM and 0.1 to 10 %, respectively.

Then, the solution capable of desorbing nucleic acids from the solid phase is introduced into the container and discharged therefrom by reducing and increasing the pressure in the same manner as described above. The discharged solution contains nucleic acids of interest. Thus, this solution is collected and utilized in the subsequent operations such as nucleic acid amplification by PCR (polymerase chain reaction).

Fig. 2 is a cross sectional view of one example of a unit for isolation and purification of nucleic acid according to the present invention, but the differential pressure generator is not shown. A container 1 containing a solid phase is composed of a main body 10 and a lid 20, and is formed by transparent polystyrene. The main body 10 contains surface-saponified cellulose triacetate membranes as solid phases 30. In addition, it has an opening 101 for sucking a sample solution and the like. A bottom face 102 running from the opening is formed in a funnel shape and provides a space 121 from a solid phase 30. A frame 103 is provided integrally with the bottom face 102 so as to maintain space 121 by supporting the solid phase 30.

The main body has an inner diameter of 20.1 mm and a depth of 5.9 mm, and the length from the bottom face 102 to the opening 101 is approximately 70 mm
Further, the solid phase 30 contained therein has a diameter of 20.0 mm and a piece of the solid phase has a thickness of approximately 50 to 500 µm. As one example, the solid phase may have a thickness of 100 µm.

In Fig. 2, a funnel-shaped depressing member 13 is provided above the solid phase. The depressing member 13 has a hole 131 in its center and also has a group of projections 132 provided downwardly, and there is provided a space 122 between the depressing member 13 and the solid phase 30. In order to prevent the sample solution and the like from leaking through a space between the solid phase 30 and a wall 104 of the main body 10, the wall 104 is prepared to have a larger diameter of its upper part than the solid phase, and the depressing member 13 has its end placed on a step 105.

The lid 20 is jointed with the main body 10 by ultrasonic heating. The lid 20 has an opening 21 almost on its center, which is used for connecting the differential pressure generator. There is provided a space 123 between the lid 20 and depressing member 13, which holds sample solution and the like flowing from the hole 131. The volume of the space 123 is approximately 0.1 ml.

### (3) Method of analyzing nucleic, acid according to the present invention

A method of analyzing nucleic acid according to the present invention comprises the steps of:
(1) isolating and purifying nucleic acid fragments containing target nucleic acids by the method of the present invention;
(2) allowing the target nucleic acid fragment, at least one primer complementary to a portion of the target nucleic acid fragment, at least one deoxynucleoside triphosphate, and at least one polymerase to react with each other, and conducting polymerase elongation reaction by using the target nucleic acid fragment as a template and using 3' terminal of the primer as an initiation site; and
(3) detecting whether polymerase elongation reaction proceeds, or whether the polymerase elongation reaction product hybridizes with another nucleic acid.

According to a preferable embodiment of the present invention, whether polymerase elongation reaction proceeds can be detected by assaying pyrophosphoric acid which is produced in accordance with polymerase elongation reaction.

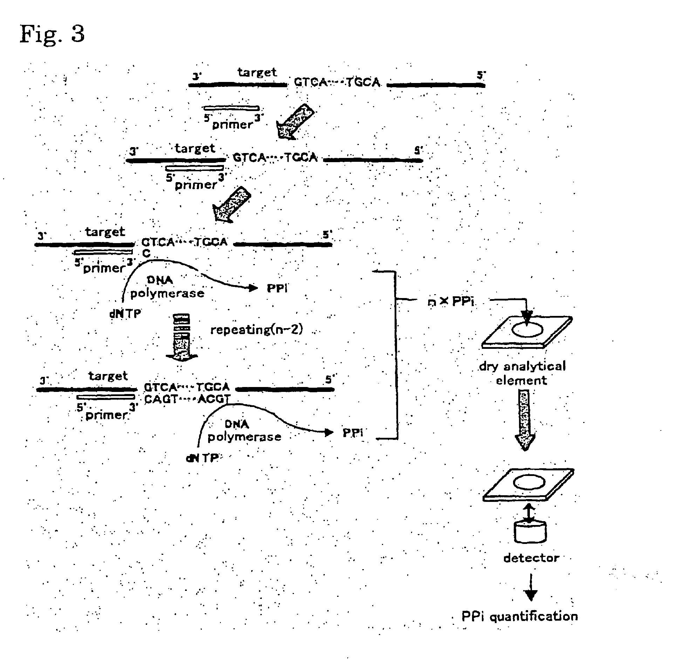

According to a further preferable embodiment, pyrophosphoric acid is analyzed by a colorimetric method, more preferably by use of a dry analytical element. According to the method of analyzing nucleic acid according to the present invention, it is possible to detect the presence or abundance of a target nucleic acid fragment, or to detect nucleotide sequences of the target nucleic acid fragment. The concept of the expression "to detect the abundance" used herein includes the quantification of the target nucleic acid fragment. Examples of the detection of nucleotide sequences of the target nucleic acid fragment include detection of mutation or polymorphism of the target nucleic acid. Fig. 3 is a schematic view illustrating an embodiment of the present invention.

A first preferable embodiment of the method of analyzing a target nucleic acid fragment according to the present invention is described hereinafter.
(a) The detection of pyrophosphoric acid is carried out using a dry analytical element for quantitative assay of pyrophosphoric acid which contains a reagent layer comprising xanthosine or inosine, pyrophosphatase, purine nucleoside phosphorylase, xanthine oxidase, peroxidase and a color developer.
(b) A polymerase used therein is one selected from the group consisting of DNA polymerase I, Klenow fragment of DNA polymerase I, Bst DNA polymerase, and reverse transcriptase.

Further, according to another embodiment of the present invention, when whether polymerase elongation reaction proceeds is determined by the detection of pyrophosporic acid which is produced in the polymerse elongation reaction, pyrophosphoric acid is enzymatically converted into inorganic phosphorus. Thereafter, for the detection of pyrophosphoric acid, used is a dry analytical method for quantitative assay of inorganic phosphorus which contains a reagent layer comprising xanthosine or inosine, purine nucleoside phosphorylase, xanthine oxidase, peroxidase and a color developer. A preferable embodiment for this case is described hereinafter.
(a) Pyrophosphatase is used as an enzyme for the conversion of pyrophosphoric acid.
(b) A polymerase used therein is one selected from the group consisting of DNA polymerase I, Klenow fragment of DNA polymerase I, Bst DNA polymerase, and reverse transcriptase.

The embodiments of the present invention will be described in more detail in the following.
(A) Target nucleic acid fragment: A target nucleic acid fragment to be analyzed in the present invention is polynucleotide, at least a part of its nucleotide sequence being known, and can be a genomic DNA fragment isolated from all the organisms including animals, microorganisms, bacteria, and plants. Also, RNA or DNA fragment which can be isolated from viruses and cDNA fragment which is synthesized using mRNA as template, can be analyzed. Preferably, the target nucleic acid fragment is purified as highly as possible, and an extra ingredient other than a nucleic acid fragment is removed. For example, when a genomic DNA fragment isolated from blood of animal (e.g., human) or nucleic acid (DNA or RNA) fragments of infectious bacteria or virus existing in blood are analyzed, cell membrane of leucocyte which was destructed in the isolation process, hemoglobin which was eluted from erythrocytes, and other general chemical substances in blood should be fully removed. In particular, hemoglobin inhibits the subsequent polymerase elongation reaction. Pyrophosphoric acid and phosphoric acid existing in blood as general biochemical substances are disturbing factors for accurate detection of pyrophosphoric acid generated by polymerase elongation reaction.
(B) Primer complementary with target nucleic acid fragment: A primer complementary with a target nucleic acid fragment used in the present invention is oligonucleotide having a nucleotide sequence complementary with a target site, the nucleotide sequence of the target nucleic acid fragment being known. Hybridization of a primer complementary with the target nucleic acid fragment to a target site of the target nucleic acid fragment results in progress on polymerase elongation reaction starting from the 3' terminus of the primer and using the target nucleic acid as template. Thus, whether or not the primer recognizes and specifically hybridizes to a target site of the target nucleic acid fragment is an important issue in the present invention. The number of nucleotides in the primer used in the present invention is preferably 5 to 60, and particularly preferably 15 to 40. If the number of nucleotides in the primer is too small, specificity with the target site of the target nucleic acid fragment is deteriorated and also a hybrid with the target nucleic acid fragment cannot be stably formed. When the number of nucleotides in the primer is too high, double-strands are disadvantageously formed due to hydrogen bonds between primers or between nucleotides in a primer. This also results in deterioration in specificity.

When the existence of the target nucleic acid fragment is detected by the method according to the present invention, a plurality of primers complementary with each different site in the target nucleic acid fragment can be used. Thus, recognition of the target nucleic acid fragment in a plurality of sites results in improvement in specificity in detecting the existence of the target nucleic acid fragment. When a part of the target nucleic acid fragment is amplified (e.g., PCR), a plurality of primers can be designed in accordance with the amplification methods.

When the nucleotide sequence of the target nucleic acid fragment is detected by the method according to the present invention, particularly when the occurrence of mutation or polymorphisms is detected, a primer is designed in accordance with a type of nucleotide corresponding to mutation or polymorphisms so as to contain a portion of mutation or polymorphisms of interest. Thus, the occurrence of mutation or polymorphisms of the target nucleic acid fragment causes difference in the occurrence of hybridization of the primer to the target nucleic acid fragment, and the detection as difference in polymerase elongation reaction eventually becomes feasible. By setting a portion corresponding to mutation or polymorphisms around the 3' terminus of the primer, difference in recognition of the polymerase reaction site occurs, and this eventually enables the detection as difference in polymerase elongation reaction.
(C) Polymerase: When the target nucleic acid is DNA, polymerase used in the present invention is DNA polymerase which catalyzes complementary elongation reaction which starts from the double-strand portion formed by hybridization of the primer with the target nucleic acid fragment in its portion denatured into single-strand in the 5' → 3' direction by using deoxynucleoside triphosphate (dNTP) as material and using the target nucleic acid fragment as template. Specific examples of DNA polymerase used include DNA polymerase I, Klenow fragment of DNA polymerase I, and Bst DNA polymerase. DNA polymerase can be selected or combined depending on the purpose. For example, when a part of the target nucleic acid fragment is amplified (e.g., PCR), use of Taq DNA polymerase which is excellent in heat resistance, is effective. When a part of the target nucleic acid fragment is amplified by using the amplification method (loop-mediated isothermal amplification of DNA (the LAMP method)) described in "BIO INDUSTRY, Vol. 18, No. 2, 2001," use of Bst DNA polymerase is effective as strand displacement-type DNA polymerase which has no nuclease activity in the 5' → 3' direction and catalyzes elongation reaction while allowing double-strand DNA to be released as single-strand DNA on the template. Use of DNA polymerase α, T4 DNA polymerase, and T7 DNA polymerase, which have hexokinase activity in the 3' → 5' direction in combination is also possible depending on the purpose.

When a genomic nucleic acid of RNA viruses or mRNA is a target nucleic acid fragment, reverse transcriptase having reverse transcription activity can be used. Further, reverse transcriptase can be used in combination with Taq DNA polymerase.
(D) Polymerase elongation reaction: Polymerase elongation reaction in the present invention includes all the complementary elongation reaction of nucleic acids which proceeds by starting from the 3' terminus of a primer complementary with the target nucleic acid fragment as described in (B) above which was specifically hybridized with a part of the portion denatured into a single-strand of the target nucleic acid fragment as described in (A), using deoxynucleoside triphosphate (dNTP) as material, using a polymerase as described in (C) above as a catalyst, and using a target nucleic acid fragment as template. This complementary nucleic acid elongation reaction indicates that continuous elongation reaction occurs at least twice (corresponding to 2 nucleotides).

Examples of a representative polymerase elongation reaction and an amplification reaction of a subject site of the target nucleic acid fragment involving polymerase elongation reaction are shown below. The simplest case is that only one polymerase elongation reaction in the 5' → 3' direction is carried out using the target nucleic acid fragment as template. This polymerase elongation reaction can be carried out under isothermal conditions. In this case, the amount of pyrophosphoric acid generated as a result of polymerase elongation reaction is in proportion to the initial amount of the target nucleic acid fragment. Specifically, it is a suitable method for quantitatively detecting the existence of the target nucleic acid fragment.

When the amount of the target nucleic acid is small, a target site of the target nucleic acid is preferably amplified by any means utilizing polymerase elongation reaction. In the amplification of the target nucleic acid, various methods which have been heretofore developed, can be used. The most general and spread method for amplifying the target nucleic acid is polymerase chain reaction (PCR). PCR is a method of amplifying a target portion of the target nucleic acid fragment by repeating periodical processes of denaturing (a step of denaturing a nucleic acid fragment from double-strand to single-strand) → annealing (a step of hybridizing a primer to a nucleic acid fragment denatured into single-strand) → polymerase (Taq DNA polymerase) elongation reaction → denaturing, by periodically controlling the increase and decrease in temperature of the reaction solution. Finally, the target site of the target nucleic acid fragment can be amplified 1,000,000 times as compared to the initial amount. Thus, the amount of accumulated pyrophosphoric acid generated upon polymerase elongation reaction in the amplification process in PCR becomes large, and thereby the detection becomes easy.

A cycling assay method using exonuclease described in Japanese Patent Publication Laying-Open No. 5-130870 is a method for amplifying a target site of the target nucleic acid fragment utilizing polymerase elongation. In this method, a primer is decomposed from a reverse direction by performing polymerase elongation reaction starting from a primer specifically hybridized with a target site of the target nucleic acid fragment, and allowing 5' → 3' exonuclease to act. In place of the decomposed primer, a new primer is hybridized, and elongation reaction by DNA polymerase proceeds again. This elongation reaction by polymerase and the decomposition reaction by exonuclease for removing the previously elongated strand are successively and periodically repeated. The elongation reaction by polymerase and the decomposition reaction by exonuclease can be carried out under isothermal conditions. The amount of accumulated pyrophosphoric acid generated in polymerase elongation reaction repeated in this cycling assay method becomes large, and the detection becomes easy.

The LAMP method is a recently developed method for amplifying a target site of the target nucleic acid fragment. This method is carried out by using at least 4 types of primers, which complimentarily recognize at least 6 specific sites of the target nucleic acid fragment, and strand displacement-type Bst DNA polymerase, which has no nuclease activity in the 5' → 3' direction and which catalyzes elongation reaction while allowing the double-strand DNA on the template to be released as single-strand DNA.
In this method, a target site of the target nucleic acid fragment is amplified as a special structure under isothermal conditions. The amplification efficiency of the LAMP method is high, and the amount of accumulated pyrophosphoric acid generated upon polymerase elongation reaction is very large, and the detection becomes easy.

When the target nucleic acid fragment is a RNA fragment, elongation reaction can be carried out by using reverse transcriptase having reverse transcription activity and using the RNA strand as template. Further, RT-PCR can be utilized where reverse transcriptase is used in combination with Taq DNA polymerase, and reverse transcription (RT) reaction is carried out, followed by PCR. Detection of pyrophosphoric acid generated in the RT reaction or RT-PCR reaction enables the detection of the existence of the RNA fragment of the target nucleic acid fragment.
This method is effective when the existence of RNA viruses is detected.
(E) Detection of pyrophosphoric acid (PPi): A method represented by formula 1 has been heretofore known as a method for detecting pyrophosphoric acid (PPi). In this method, pyrophosphoric acid (PPi) is converted into adenosinetriphosphate (ATP) with the aid of sulfurylase, and luminescence generated when adenosinetriphosphate acts on luciferin with the aid of luciferase is detected. Thus, an apparatus capable of measuring luminescence is required for detecting pyrophosphoric acid (PPi) by this method.

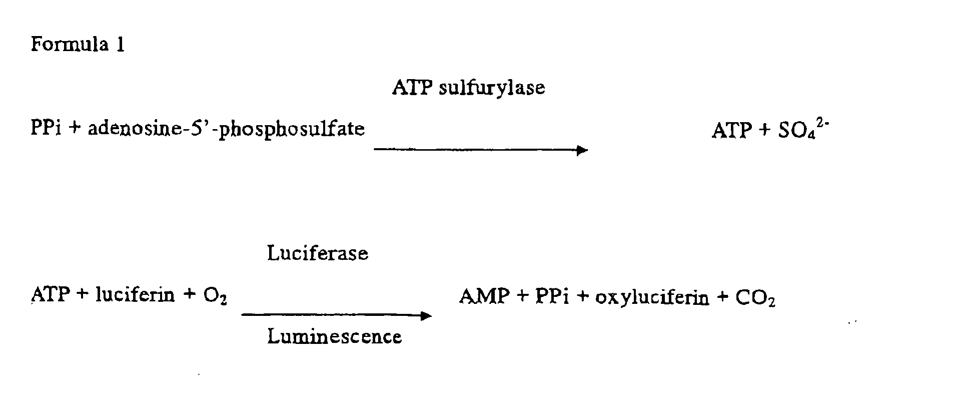

A method for detecting pyrophosphoric acid suitable for the present invention is a method represented by formula 2 or 3. In the method represented by formula 2 or 3, pyrophosphoric acid (PPi) is converted into inorganic phosphate (Pi) with the aid of pyrophosphatase, inorganic phosphate (Pi) is reacted with xanthosine or inosine with the aid of purine nucleoside phosphorylase (PNP), the resulting xanthine or hypoxanthine is oxidated with the aid of xanthine oxidase (XOD) to generate uric acid, and a color developer (a dye precursor) is allowed to develop color with the aid of peroxidase (POD) using hydrogen peroxide (H₂O₂) generated in the oxidation process, followed by colorimetry. In the method represented by formula 2 or 3, the result can be detected by colorimetry and, thus, pyrophosphoric acid (PPi) can be detected visually or using a simple colorimetric measuring apparatus.

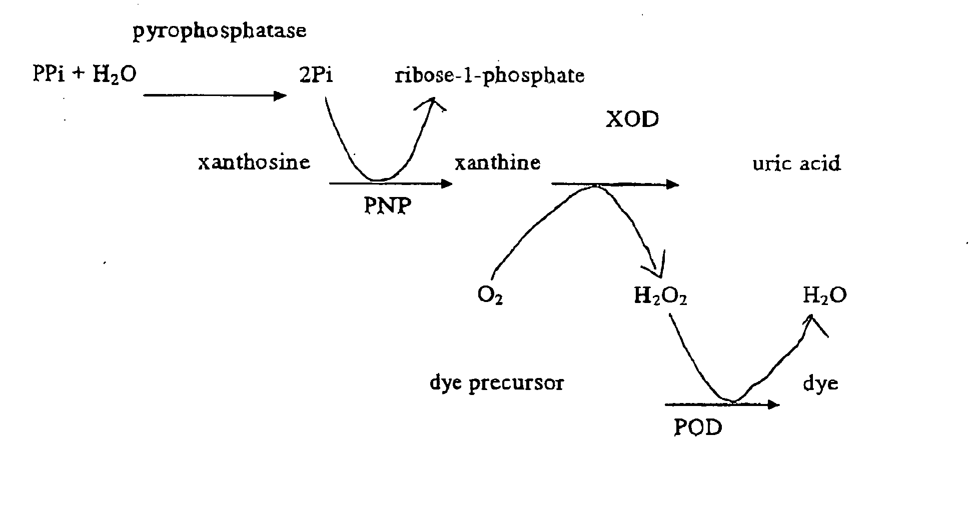

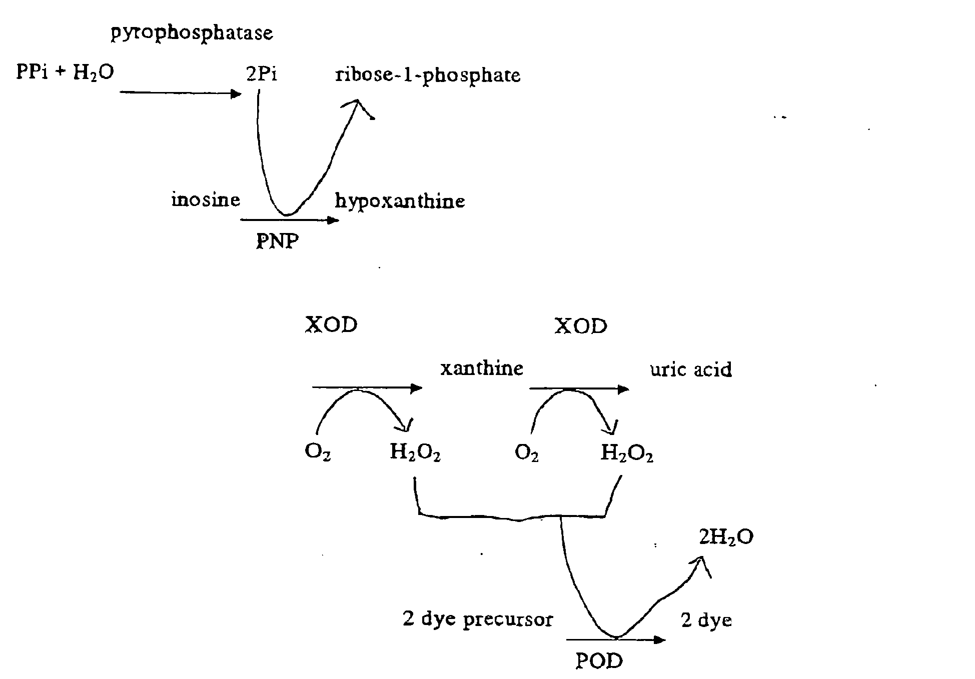

Commercially available pyrophosphatase (EC3, 6, 1, 1), purine nucleoside phosphorylase (PNP, EC2. 4. 2. 1), xanthine oxidase (XOD, EC1. 2. 3. 2), and peroxidase (POD, EC1. 11. 1. 7) can be used. A color developer (i.e., a dye precursor) may be any one as long as it can generate a dye by hydrogen peroxide and peroxidase (POD), and examples thereof which can be used herein include: a composition which generates a dye upon oxidation of leuco dye (e.g., triarylimidazole leuco dye described in U.S.Patent. No. 4,089,747 and the like, diarylimidazole leuco dye described in Japanese Patent Publication Laying-Open No. 59-193352 (EP 0122641A)); and a composition (e.g., 4-aminoantipyrines and phenols or naphthols) containing a compound generating a dye by coupling with other compound upon oxidation.
(F) Dry analytical element: A dry analytical element which can be used in the present invention is an analytical element which comprises a single or a plurality of functional layers, wherein at least one layer (or a plurality of layers) comprises a detection reagent, and a dye generated upon reaction in the layer is subjected to quantification by colorimetry by reflected light or transmitted light from the outside of the analytical element.

In order to perform quantitative analysis using such a dry analytical element, a given amount of liquid sample is spotted onto the surface of a developing layer. The liquid sample spread on the developing layer reaches the reagent layer and reacts with the reagent thereon and develops color. After spotting, the dry analytical element is maintained for a suitable period of time at given temperature (for incubation) and a color developing reaction is allowed to thoroughly proceed. Thereafter, the reagent layer is irradiated with an illuminating light from, for example, a transparent support side, the amount of reflected light in a specific wavelength region is measured to determine the optical density of reflection, and quantitative analysis is carried out based on the previously determined calibration curve.

Since a dry analytical element is stored and kept in a dry state before detection, it is not necessary that a reagent is prepared for each use. As stability of the reagent is generally higher in a dry state, it is better than a so-called wet process in terms of simplicity and swiftness since the wet process requires the preparation of the reagent solution for each use. It is also excellent as an examination method because highly accurate examination can be swiftly carried out with a very small amount of liquid sample.
(G) Dry analytical element for quantifying pyrophosphoric acid: A dry analytical element for quantifying pyrophosphoric acid which can be used in the present invention can have a layer construction which is similar to various known dry analytical elements. The dry analytical element may be multiple layers which contain, in addition to a reagent for performing the reaction represented by formula 2 or 3 according to item (E) above (detection of pyrophosphoric acid (PPi)), a support, a developing layer, a detection layer, a light-shielding layer, an adhesive layer, a water-absorption layer, an undercoating layer, and other layers. Examples of such dry analytical elements include those disclosed in the specifications of Japanese Patent Publication Laying-Open No. 49-53888 (U.S. Patent. No. 3,992,158), Japanese Patent Publication Laying-Open No. 51-40191 (U.S. Patent. No. 4,042,335), Japanese Patent Publication Laying-Open No. 55-164356 (U.S Patent. No. 4,292,272), and Japanese Patent Publication Laying-Open No. 61-4959 (EPC Publication No. 0166365A).

Examples of the dry analytical element to be used in the present invention include a dry analytical element for quantitative assay of pyrophosphoric acid which has a reagent layer comprising a reagent which converts pyrophosphoric acid into inorganic phosphorus, and a group of reagents capable of color reaction depending on the amount of inorganic phosphorus.

In this dry analytical element for quantitative assay of pyrophosphate, pyrophosphoric acid (PPi) can enzymatically be converted into inorganic phosphorus (Pi) using pyrophosphatase as described above. The subsequent process, that is color reaction depending on the amount of inorganic phosphorus (Pi), can be performed using "quantitative assay method of inorganic phosphorus" (and combinations of individual reactions used therefor), described hereinafter, which is known in the field of biochemical inspection.

It is noted that when representing "inorganic phosphorus," both the expressions "Pi" and "HPO₄²⁻, H₂PO₄¹⁻' are used for phosphoric acid (phosphate ion). Although the expression "Pi" is used in the examples of reactions described below, the expression "HPO₄²⁻" may be used for the same reaction formula.

As the quantitative assay method of inorganic phosphorus, an enzyme method and a phosphomolybdate method are known. Hereinafter, this enzyme method and phosphomolybdate method will be described as the quantitative assay method of inorganic phosphorus.

### A. Enzyme method

Depending on the enzyme to be used for the last color reaction during a series of reactions for Pi quantitative detection, the following methods for quantitative assay are available: using peroxidase (POD); or using glucose-6-phosphate dehydrogenase (G6PDH), respectively. Hereinafter, examples of these methods are described.

### (1) Example of the method using peroxidase (POD)

### (1-1)

Inorganic phosphorus (Pi) is allowed to react with inosine by purine nucleoside phosphorylase (PNP), and the resultant hypoxanthine is oxidized by xanthine oxidase (XOD) to produce uric acid. During this oxidization process, hydrogen peroxide (H₂O₂) is produced. Using the thus produced hydrogen peroxide, 4-aminoantipyrines (4-AA) and phenols are subjected to oxidization-condensation by peroxidase (POD) to form a quinonimine dye, which is colorimetrically assessed.

### (1-2)

Pyruvic acid is oxidized by pyruvic oxidase (POP) in the presence of inorganic phosphorus (Pi), cocarboxylase (TPP), flavin adenine dinucleotide (FAD) and Mg²⁺ to produce acetyl acetate. During this oxidization process, hydrogen peroxide (H₂O₂) is produced. Using the thus produced hydrogen peroxide, 4-aminoantipyrines (4-AA) and phenols are subjected to oxidization-condensation by peroxidase (POD) to form a quinonimine dye which is colorimetrically assessed, in the same manner as described in (1-1).

It is noted that the last color reaction for each of the above processes (1-1) and (1-2) can be performed by a "Trinder reagent" which is known as a detection reagent for hydrogen peroxide. In this reaction, phenols function as "hydrogen donors." Phenols to be used as "hydrogen donors" are classical, and now various modified "hydrogen donors" are used. Examples of these hydrogen donors include N-ethyl-N-sulfopropyl-m-anilidine, N-ethyl-N-sulfopropylaniline, N-ethyl-N-sulfopropyl-3,5-dimethoxyaniline, N-sulfopropyl-3,5-dimethoxyaniline, N-ethyl-N-sulfopropyl-3,5-dimethylaniline, N-ethyl-N-sulfopropyl-m-toluidine, N-ethyl-N-(2-hydroxy-3-sulfopropyl)-m-anilidine, N-ethyl-N-(2-hydroxy-3-sulfopropyl) aniline, N-ethyl-N-(2-hydroxy-3-sulfopropyl)-3,5-dimethoxyaniline, N-(2-hydroxy-3-sulfopropyl)-3,5-dimethoxyaniline, N-ethyl-N-(2-hydroxy-3-sulfopropyl)-3,5-dimethylaniline N-ethyl-N-(2-bydroxy-3-sulfopropyl)-m-toluidine, and N-sulfopropylaniline.

### (2) Example of a method using glucose-6-phosphate dehydrogenase (G6PDH)

### (2-1)

Inorganic phosphorus (Pi) is reacted with glycogen with phosphorylase to produce glucose-1-phosphate (G-1-P). The produced glucose-1-phosphate is converted into glucose-6-phosphate (G-6-P) with phosphoglucomutase (PGM). In the presence of glucose-6-phosphate and nicotiamide adenine dinucleotide (NAD), NAD is reduced to NADH with glucose-6-phosphate dehydrogenase (G6PDH), followed by colorimetric analysis of the produced NADH.

### (2-2)

Inorganic phosphorus (Pi) is reacted with maltose with maltose phosphorylase (MP) to produce glucose-1-phosphate (G-1-P). Thereafter, the produced glucose-1-phosphate is converted into glucose-6-phosphate (G-6-P) with phosphoglucomutase (PGM) in the same manner as described in (2-1). In the presence of glucose-6-phosphate and nicotiamide adenine dinucleotide (NAD), NAD is reduced to NADH, with glucose-6-phosphate dehydrogenase (G6PDH), followed by colorimetric analysis of the produced NADH.

### B. Phosphomolybdate method

There are two phosphomolybdate methods. One is a direct method wherein "Phosphomolybdates (H₃[PO₄Mo₁₂O₃₆])" prepared by complexing inorganic phosphorus (phosphate) and aqueous molybdate ions under acidic condition are directly quantified. The other is a reduction method wherein further to the above direct method, Mo(IV) is reduced to Mo(III) by a reducing agent and molybudenum blue (Mo(III)) is quantified. Examples of the aqueous molybdate ions include aluminum molybdate, cadmium molybdate, calcium molybdate, barium molybdate, lithium molybdate, potassium molybdate, sodium molybdate, and ammonium molybdate. Representative examples of the reducing agents to be used in the reduction method include 1-amino-2-naphthol-4-sulfonic acid, ammonium ferrous sulfate, ferrous chloride, stannous chloride-hydrazine, p-methylarainophenol sulfate, N,N'-dimethyl-phenylenediamine, ascorbic acid, and malachite green.

When a light-transmissive and water-impervious support is used, the dry analytical element can be practically constructed as below. However, the scope of the present invention is not limited to these.
(1) One having a reagent layer on the support.
(2) One having a detection layer and a reagent layer in that order on the support.
(3) One having a detection layer, a light reflection layer, and a reagent layer in that order on the support
(4) One having a second reagent layer, a light reflection layer, and a first reagent layer in that order on the support.
(5) One having a detection layer, a second reagent layer, a light reflection layer, and a first reagent layer in that order on the support.

In (1) to (3) above, the reagent layer may be constituted by a plurality of different layers. For example, a first reagent layer may contain enzyme pyrophosphatase which is required in the pyrophosphatase reaction represented by formula 2 or 3, and substrate xanthosine or substrate inosine and enzyme PNP which are required in the PNP reaction, a second reagent layer may contain enzyme XOD which is required in the XOD reaction represented by formula 2 or 3, and a third reagent layer may contain enzyme POD which is required in the POD reaction represented by formula 2 or 3, and a coloring dye (dye precursor). Alternatively, two reagent layers are provided- On the first reagent layer, the pyrophosphatase reaction and the PNP reaction may be proceeded, and the XOD reaction and the POD reaction may be proceeded on the second reagent layer. Alternatively, the pyrophosphatase reaction, the PNP reaction and the XOD reaction may be proceeded on the first reagent layer, and the POD reaction may be proceeded on the second reagent layer.

A water absorption layer may be provided between a support and a reagent layer or detection layer. A filter layer may be provided between each layer. A developing layer may be provided on the reagent layer and an adhesive layer may be provided therebetween.

Any of light-nontransmissive (opaque), light-semitransmissive (translucent), or light-transmissive (transparent) support can be used. In general, a light-transmissive and water-impervious support is preferred. Preferable materials for a light-transmissive and water-impervious support are polyethylene terephthalate or polystyrene. In order to firmly adhere a hydrophilic layer, an undercoating layer is generally provided or hydrophilization is carried out.

When a porous layer is used as a reagent layer, the porous medium may be a fibrous or nonfibrous substance. Fibrous substances used herein include, for example, filter paper, non-woven fabric, textile fabric (e.g. plain-woven fabric), knitted fabric (e.g., tricot knitted fabric), and glass fiber filter paper. Nonfibrous substances may be any of a membrane filter comprising cellulose acetate etc., described in Japanese Patent Publication Laying-Open No. 49-53888, or a particulate structure having mutually interconnected spaces comprising fine particles of inorganic substances or organic substances described in, for example, Japanese Patent Publication Laying-Open No. 49-53888, Japanese Patent Publication Laying-Open No. 55-90859 (U.S. Patent. No. 4,258,001), and Japanese Patent Publication Laying-Open No. 58-70163 (U.S. Patent. No. 4,486,537). A partially-adhered laminate which comprises a plurality of porous layers described in, for example, Japanese Patent Publication Laying-Open No. 61-4959 (EP Publication 0166365A), Japanese Patent Publication Laying-Open No. 62-116258, Japanese Patent Publication. Laying-Open No. 62-138756 (EP Publication 0226465A), Japanese Patent Publication Laying-Open No. 62-138757 (EP Publication 0226465A), and Japanese Patent Publication Laying-Open No. 62-138758 (EP Publication 0226465A), is also preferred.

A porous layer may be a developing layer having so-called measuring action, which spreads liquid in an area substantially in proportion to the amount of the liquid to be supplied. Preferably, a developing layer is textile fabric, knitted fabric, and the like. Textile fabrics and the like may be subjected to glow discharge treatment as described in Japanese Patent Publication Laying-Open No. 57-66359. A developing layer may comprise hydrophilic polymers or surfactants as described in Japanese Patent Publication Laying-Open No. 60-222770 (EP 0162301A), Japanese Patent Publication Laying-Open No. 63-219397 (German Publication DE 3717913A), Japanese Patent Publication Laying-Open No. 63-112999 (DE 3717913A), and Japanese Patent Publication Laying-Open No. 62-182652 (DE 3717913A) in order to regulate a developing area, a developing speed and the like.

For example, a method is useful where the reagent of the present invention is previously impregnated into or coated on a porous membrane etc., comprising paper, fabric or polymer, followed by adhesion onto another water-pervious layer provided on a support (e.g., a detection layer) by the method as described in Japanese Patent Publication Laying-Open No. 55-1645356.

The thickness of the reagent layer thus prepared is not particularly limited.
When it is provided as a coating layer, the thickness is suitably in the range of about 1 µ m to 50 µ m, preferably in the range of 2 µ m to 30 µ m. When the reagent layer is provided by a method other than coating, such as lamination, the thickness can be significantly varied in the range of several tens of to several hundred µ m.

When a reagent layer is constituted by a water-pervious layer of hydrophilic polymer binders, examples of hydrophilic polymers which can be used include: gelatin and a derivative thereof (e.g., phthalated gelatin); a cellulose derivative (e.g., hydroxyethyl cellulose); agarose, sodium arginate; an acrylamide copolymer or a methacrylamide copolymer (e.g., a copolymer of acrylamide or methacrylamide and various vinyl monomers); polyhydroxyethyl methacrylate; polyvinyl alcohol; polyvinyl pyrrolidone; sodium polyacrylate; and a copolymer of acrylic acid and various vinyl monomers.

A reagent layer composed of hydrophilic polymer binders can be provided by coating an aqueous solution or water dispersion containing the reagent composition of the present invention and hydrophilic polymers on the support or another layer such as a detection layer followed by drying the coating in accordance with the methods described in the specifications of Japanese Patent Examined Publication No. 53-21677 (U.S. Patent No. 3,992,158), Japanese Patent Publication Laying-Open No. 55-164356 (U.S. Patent. No. 4,292,272), Japanese Patent Publication Laying-Open No. 54-101398 (U.S. Patent. No. 4,132,528). The thickness of the reagent layer comprising hydrophilic polymers as binders is about 2 µ m to about 50 µm, preferably about 4 µ m to about µ m on a dry basis, and the coverage is about 2 g/m² to about 50 g/m², preferably about 4 g/m² to about 30 g/m².

The reagent layer can further comprise an enzyme activator, a coenzyme, a surfactant, a pH buffer composition, an impalpable powder, an antioxidant, and various additives comprising organic or inorganic substances in addition to the reagent composition represented by formula 2 or 3 in order to improve coating properties and other various properties of diffusible compounds such as diffusibility, reactivity, and storage properties. Examples of buffers which can be contained in the reagent layer include pH buffer systems described in "Kagaku Binran Kiso (Handbook on Chemistry, Basic)," The Chemical Society of Japan (ed.), Maruzen Co., Ltd. (1996), p.1312-1320, "Data for Biochemical Research, Second Edition, R. M. C. Dawson et al. (2nd ed.), Oxford, at the Clarendon Press (1969), p. 476-508, "Biochemistry" 5, p. 467-477 (1966), and "Analytical Biochemistry" 104, p. 300-310 (1980). Specific examples of pH buffer systems include a buffer containing borate; a buffer containing citric acid or citrate; a buffer containing glycine, a buffer containing bicine; a buffer containing HEPES; and Good's buffers such as a buffer containing MES. A buffer containing phosphate cannot be used for a dry analytical element for detecting pyrophosphoric acid.

The dry analytical element for quantifying pyrophosphoric acid which can be used in the present invention can be prepared in accordance with a known method disclosed in the above-described various patent specifications. The dry analytical element for quantifying pyrophosphoric acid is cut into small fragments, such as, an about 5 mm to about 30 mm-square or a circle having substantially the same size, accommodated in the slide frame described in, for example, Japanese Patent Examined Publication No. 57-283331 (U.S. Patent. No. 4,169,751), Japanese Utility Model Publication Laying-Open No. 56-142454 (U.S. Patent. No. 4,387,990), Japanese Patent Publication Laying-Open No. 57-63452, Japanese Utility Model Publication Laying-Open No. 58-32350, and Japanese Patent Publication Laying-Open No. 58-501144 (International Publication WO 083/00391), and used as slides for chemical analysis. This is preferable from the viewpoints of production, packaging, transportation, storage, measuring operation, and the like. Depending on its intended use, the analytical element can be accommodated as a long tape in a cassette or magazine, as small pieces accommodated in a container having an opening, as small pieces applied onto or accommodated in an open card, or as small pieces cut to be used in that state.

The dry analytical element for quantifying pyrophosphoric acid which can be used in the present invention can quantitatively detect pyrophosphoric acid which is a test substance in a liquid sample, by operations similar to that described in the above-described patent specifications and the like. For example, about 2 µ L to about 30 µ L, preferably 4 µ L to 15 µ L of aqueous liquid sample solution is spotted on the reagent layer. The spotted analytical element is incubated at constant temperature of about 20°C to about 45°C; preferably about 30°C to about 40°C for 1 to 10 minutes. Coloring or discoloration in the analytical element is measured by the reflection from the light-transmissive support side, and the amount of pyrophosphoric acid in the specimen can be determined based on the principle of colorimetry using the previously prepared calibration curve. Quantitative analysis can be carried out with high accuracy by keeping the amount of liquid sample to be spotted, the incubation time, and the temperate at constant levels..

Quantitative analysis can be carried out with high accuracy in a very simple operation using chemical analyzers described in, for example, Japanese Patent Publication Laying-Open No. 60-125543, Japanese Patent Publication Laying-Open No. 60-220862, Japanese Patent Publication Laying-Open No. 61-294367, and Japanese Patent Publication Laying-Open No. 58-161867 (U.S. Patent. No.4,424,191). Semiquantitative measurement may be carried out by visually judging the level of coloring depending on the purpose and accuracy needed.

Since the dry analytical element for quantifying pyrophosphoric acid which can be used in the present invention is stored and kept in a dry state before analysis, it is not necessary that a reagent is prepared for each use, and stability of the reagent is generally higher in a dry state. Thus, in terms of simplicity and swiftness, it is better than a so-called wet process, which requires the preparation of the reagent solution for each use. It is also excellent as an examination method because highly accurate examination can be swiftly carried out with a very small amount of liquid sample.

The dry analytical element for quantifying inorganic phosphorus which can be used in the second aspect of the present invention can be prepared by removing pyrophosphatase from the reagent layer in the aforementioned dry analytical element for quantifying pyrophosphoric acid. The dry analytical element described in Japanese Patent Publication Laying-Open No. 7-197 can also be used. The dry analytical element for quantifying inorganic phosphorus is similar to the aforementioned dry analytical element for quantifying pyrophosphoric acid in its layer construction, method of production, and method of application, with the exception that the reagent layer does not comprise pyrophosphatase.
(H) Kit: The analysis of the target nucleic acid according to the present invention can be analyzed using a kit comprising at least one primer complementary with a part of the target nucleic acid fragment to be analyzed, at least one deoxynucleoside triphosphate (dNTP), at least one polymerase, and a dry analytical element for quantifying pyrophosphoric acid.

The form of the kit may be a cartridge comprising: an opening capable of supplying a liquid containing the target nucleic acid fragment, at least a part of its nucleotide sequence being known; at least one primer complementary with a part of the target nucleic acid fragment; at least one deoxynucleoside triphosphate (dNTP), at least one reaction cell unit capable of retaining at least one polymerase; a detection unit capable of retaining a dry analytical element for quantifying pyrophosphoric acid; and a canaliculus or groove capable of connecting the opening, the reaction cell unit, and the detection unit and transferring liquid among them

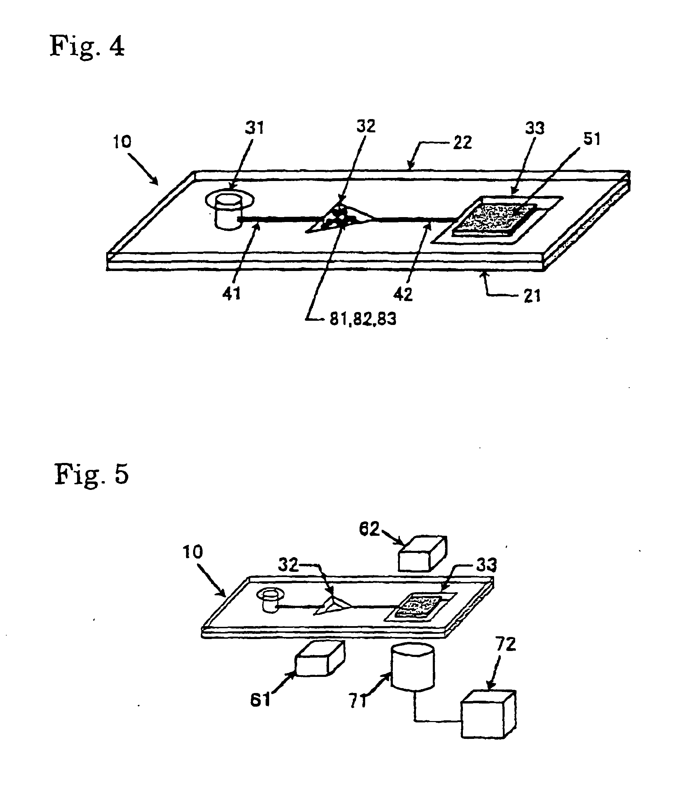

The cartridge disclosed in U.S. Patent No. 5,919,711 can be used as such a cartridge. An embodiment of a kit according to the present invention in the form of a cartridge was shown in Fig. 4. In kit a sample liquid containing the target nucleic acid can be supplied from opening 31. Opening 31 is connected to reaction cell 32 through canaliculus 41. Reaction cell 32 maintains in advance at least one primer 81 complementary with a part of the target nucleic acid fragment, at least one deoxynucleoside triphosphate (dNTP) 82, and at least one polymerase 83. Reaction cell 32 is further connected to detection unit 33 through canaliculus 42. Detection unit 33 maintains in advance dry analytical element 51. The sample solution, in which polymerase elongation reaction has proceeded in reaction cell 32, is transferred through canaliculus 42, supplied on dry analytical element 51 for quantifying pyrophosphoric acid in detection unit 33, and detects pyrophosphoric acid generated by polymerase elongation reaction. In kit 10, liquid transference between opening 31 and reaction cell 32 and between reaction cell 32 and detection unit 33 can be carried out by centrifuge force, electrophoresis, electroosmosis, or the like. Preferably, reaction cell 32, canalic-pluses 41 and 42, and detection unit 33 are hermetically sealed with base body 21 and lid 22.

When kit 10 in the form of cartridge as shown in Fig. 4 is used, as shown in Fig. 5, an apparatus which comprises temperature control units 61 and 62 of reaction cell 32 and detection unit 33 and detection units 71 and 72 capable of detecting coloring or color change in dry analytical element 51 for quantifying pyrophosphoric acid by reflection light, is preferably used in combination.

The kit in the form of cartridge which can be used in the present invention is not limited to those shown in Fig. 4. Reagents required in polymerase elongation reaction may be respectively retained in separate spaces. In that case, each reagent may be transferred to a reaction cell at the time of reaction. There may be a plurality of reaction cells.

When pyrophosphoric acid generated in polymerase elongation reaction is detected by enzymatically converting pyrophosphoric acid into inorganic phosphoric acid, followed by the use of dry analytical element for quantifying inorganic phosphorus, at least one primer complementary with a part of the target nucleic acid fragment, at least one deoxynucleoside triphosphate (dNTP), and at least one polymerase are previously retained in the first reaction cell, and polymerase elongation reaction is carried out in the first reaction cell. Subsequently, the reaction solution generated in the first reaction cell is transferred to the second reaction cell, which is connected to the first reaction cell through a canaliculus and already holding pyrophosphatase, pyrophosphoric acid generated in polymerase elongation reaction in the first reaction cell is converted into inorganic phosphoric acid in the second reaction cell. The reaction solution in the second reaction cell is then transferred to the detection unit, which is connected to the second reaction cell through a canaliculus and previously retains a dry analytical element for quantifying inorganic phosphorus, thereby detecting inorganic phosphorus.

A set of "opening - canaliculus - reaction cell - canaliculus - detection unit" is arranged in parallel on one cartridge, or plural sets thereof can be arranged in concentric circles in the radius direction. In this case, for example, the nucleotide sequence of at least one primer complementary with a part of the target nucleic acid fragment retained in the reaction cell can be modified in accordance with the type of the targeted nucleic acid to provide a kit capable of simultaneously detecting a plurality of target nucleic acids.

### (4) Analytical apparatus of the present invention

Further, the present invention provides an analytical apparatus for conducting nucleic acid analyses as described above. The analytical apparatus comprises:
(1) means for extracting and purifying a nucleic acid, which contains the unit for isolation and purification of nucleic acid described above in the present specification;
(2) reaction means for conducting polymerase elongation reaction; and
(3) means for detecting whether polymerase elongation reaction proceeds, or whether the polymerase elongation reaction product hybridizes with other nucleic acids.

The means for extracting and purifying a nucleic acid comprises the unit for isolation and purification of nucleic acid as described hereinabove, and further comprises a space for setting a sample liquid, a space for setting the unit for isolation and purification of nucleic acid, means for incubating the sample at a constant temperature (e.g., 37°C), and means for sucking and discharging the sample liquid or treated liquid.

The reaction means for conducting polymerase elongation reaction is means which allows nucleic acid synthesis reactions such as PCR to be conducted, and it is generally composed of a reaction container for conducting the reaction and dispensing means for adding reagents necessary for the reaction into the reaction container. In addition, it may have temperature adjusting means (e.g., a thermal cycler or an incubator) for adjusting the temperature inside the reaction container. By the dispensing means, a nucleic acid fragment comprising a target nucleic acid fragment, which is purified by the unit for isolation and purification of nucleic acid, at least one primer complementary to a portion of the target nucleic acid fragment, at least one deoxynucleoside triphosphate, and at least one polymerase, are added to the reaction container, and polymerase elongation reaction is performed with using the target nucleic acid fragment as a template and using the primer 3' terminal as an initiation site.

As the means for detecting whether polymerase elongation reaction proceeds, a dry analytical element for quantitative assay of pyrophosphoric acid or a dry analytical element for quantitative assay of inorganic phosphorus, as described hereinabove, can be used. Further to these, means for incubating the dry analytical element at a constant temperature and a reflection photometer for measuring the coloring of the dry analytical element can also be provided. Furthermore, as means for detecting whether a polymerase elongation reaction product is hybridized with other nucleic acid, any means which can commonly be used for the detection of presence or absence of hybridization can be utilized.

The analytical apparatus (particularly in the case of using analytical element for quantitative assay of pyrophosphoric acid) of the present invention can use all of (or some of) the nucleic acid aqueous solution which has been isolated and purified for the following process of polymerase elongation reaction, and thereafter can use all of (or some of) the reaction solution after polymerase elongation reaction for the following detection process by the analytical element for quantitative assay of pyrophosphoric acid (that is, the reaction solution after polymerase elongation reaction can be dispensed as a spot on an analytical element for quantitative assay of pyrophosphoric acid), and therefore the apparatus is very suitable for system automation.

The present invention will hereinafter be described in detail by Examples, but the present invention is not limited to these Examples.

### Examples

### Example 1

### (1) Preparation of a container for unit for isolation and purification of nucleic acid

A container for a unit for isolation and purification of nucleic acid, which has with a portion for containing a solid phase for nucleic acid adsorption having an inner diameter of 7 mm and a thickness of 2 mm, was made of high-impact polystyrene

### (2) Preparation of nucleic acid purification solid phases and unit for isolation and purification of nucleic acid s

As shown in Table 1, nucleic acid purification solid phases and comparative solid phases were prepared. For surface-saponification, materials to be surface-saponified were soaked in 0.02 N to 2 N sodium hydroxide aqueous solution for 20 minutes. The surface saponification ratio are varied depending on the concentration of sodium hydroxide. These solid phases were each contained into the solid phase accommodation portion of the container for unit for isolation and purification of nucleic acid in an amount as indicated in Table 1, thereby preparing a unit for isolation and purification of nucleic acid.

**Table 1**

| Properties of prepared solid phases (solid phases A, B, C, F, G, H, I are for comparative purpose) | | | |
|---|---|---|---|
| Solid phase | Material | Surface saponification ratio | No. of solid phases or shape |
| Solid phase A | Microfilter FM500* | 0% | 1 piece |
| Solid phases B | Surface-saponified solid phase A | 5% | 1 piece |
| Solid phase C | Surface-saponified Solid phase A | 10% | piece |
| Solid phase D | Surface-saponified solid phase A | 50% | 1 piece |
| Solid phase E | Surface-saponified solid phase A | 100% | 1 piece |
| Solid phase F | Cellulose triacetate base** | 0% | 0.5 mm φ of chip |
| Solid phase G | Surface-saponified solid phase F | 80% | 0.5 mm φ of chip |
| Solid phase H | polyethylene beads*** coated with cellulose triacetate | 0% | 0.3 mm φ of beads |
| Solid phase I | Surface-saponified solid phase H | 70% | 0:3 mm φ of beads |

| | | | |
|---|---|---|---|
| * porous cellulose triacetate membrane manufactured by Fuji Photo Film Co., Ltd. ** Cellulose triacetate base manufactured by Fuji Photo Film Co., Ltd. *** manufactured by Kaneka Corporation | | | |

### (3) Reparation of a buffer solution for nucleic acid adsorption and a washing buffer solution

A buffer solution for nucleic acid adsorption and a washing buffer solution were prepared according to the formulation indicated in Table 2.

**Table 2 Formulation of adsorption buffer solution for nucleic acid purification and washing buffer solution**

| 1. Buffer solution for purifying nucleic acids | |
|---|---|
| Component | Amount |
| guanidine hydrochloride (Life Technologies, Inc.) | 382 g |
| Tris (Life Technologies, Inc.) | 12.1 g |
| Triton-X100 (ICN) | 10 g |
| Twice Distilled water | 1000 ml |

| 2. Buffer solution for washing nucleic acids | |
|---|---|
| Component | |
| 10 mM Tris-HCl 70% ethanol | |

### (4) Procedures for purifying nucleic acids

200 µl of human whole blood was collected using a vacuum blood collecting tube. To this, 2.00 µl of buffer solution for nucleic acid adsorption prepared as indicated in Table 2 and 20 µl of protease K were added, and the mixture was incubated for 10 minutes at 60°C. After incubation, 200 µl of ethanol was added and the mixture was stirred. After stirring, a tip of disposable pipette connected to one opening of a unit for isolation and purification of nucleic acid prepared in Process (1) was inserted into the whole blood sample as treated above, and the sample solution was sucked and discharged using an injector connected to the other opening of the unit for isolation and purification of nucleic acid.

Immediately after discharging, the pipette tip was inserted into 1 ml of the buffer solution for washing nucleic acid, and the solution was sucked and discharged to wash the inside of the unit for isolation and purification of nucleic acid. After washing, the pipette tip was inserted into 200 µl of purified distilled water, and the distilled water was sucked and discharged. Then, the discharged solution was collected.

### (5) Quantitative determination of nucleic acid yield, and purity determination thereof

The absorbance of the collected discharged solution was measured to quantify the yield and purity of nucleic acids. The yield was quantified by measuring the absorbance at a wavelength of 260 nm, and the purity of nucleic acids was determined based on the ratio between the absorbance at 260 nm and at 280 nm. A ratio of 1.8 or more indicates good purity. The results are shown in Table 3. From these results, when the surface saponification ratio was 5% or more, DNA yield was good and the purity was high.

**Table 3**

| Used solid phases, and yield and purity of nucleic acid (solid phases A, B, C, F, G, H, I are for comparative purpose) | | | | | |
|---|---|---|---|---|---|
| Solid phase | Material | Surface saponification ratio | No. of solid phases or shape | Yield of nucleic acid (µg) | A260/A280 |
| Solid phase A | Microfilter FM500* | 0% | 1 piece | 0.1 | not measurable |
| Solid phase B | Surface-saponified solid phase A | 5% | 1 piece | 1.2 | 1.814 |
| Solid phase C | Surface-saponified solid phase A | 10% | 1 piece | 11.2 | 1.953 |
| Solid phase D | Surface-saponified solid phase A | 50% | 1 piece | 16.5 | 1.882 |
| Solid phase E | Surface-saponified solid phase A | 100% | 1 piece | 14.5 | 1.905 |
| Solid phases F | Cellulose triacetate base** | 0% | 0.5 mm φ of chip | 0 | not measurable |
| Solid phase G | Surface-saponified solid phase F | 80% | 0.5 mm φ of chip. | 5.8 | 1.898 |
| Solid phase H | Polyethylene beads*** coated with cellulose triacetate | 0% | 0.3 mm φ of beads | 0 | not measurable |
| Solid phase I | Surface-saponified solid phase H | 70% | 0.3 mm φ of beads | 7.2 | 1.803 |

| | | | | | |
|---|---|---|---|---|---|
| * porous cellulose triacetate membrane manufactured by Fuji Photo Film Co., Ltd. ** cellulose triacetate base manufactured by Fuji Photo Film Co., Ltd. *** manufactured by Kaneka Corporation | | | | | |

### Example 2: Purification of nucleic acid from whole blood sample using 100% surface-saponified porous cellulose triacetate membrane

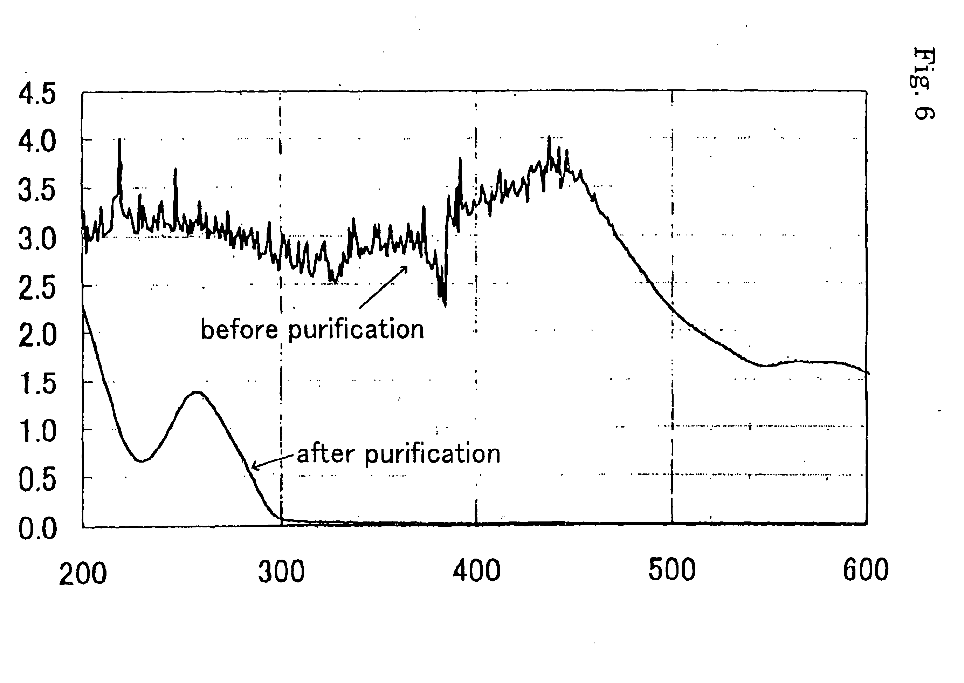

As a solid phase for adsorbing nucleic acids, 100% surface-saponified porous cellulose triacetate membrane (Fuji Photo Film Co., Ltd.)(solid phase E of Example 1) was used, and nucleic acids were purified from a whole blood sample in the same manner as Example 1. The ODs were measured on the whole blood sample before purification (OD was measured after 5-time dilution) and after purification. The results are shown in Fig. 6. From the results of Fig. 6, it is understood that components other than nucleic acids were completely removed by the isolation and purification method of the present invention.

### Example 3: Amplification of nucleic acid

The nucleic acids which were purified in Example 2 were amplified with polymerase chain reaction. As a positive control, Human DNA manufactured by CLONTECH was used. The reaction solutions for PCR were purified water (36.5 µl), 10×PCR buffer (5µl), 2.5mM of dNTP (4µl), Taq FP (0.5µl), primers (2µl), and sample (nucleic acid) (2µl).

In PCR, 30 cycles of denaturation: 94°C for 30 seconds, annealing: 65°C for 30 seconds, and elongation reaction:72°C for 1 minute were repeated. The following primers were used.
1) p53 exon 6
   Forward: GCGCTGCTCA GATAGCGATG (SEQ ID NO: 9)
   Reverse: GGAGGGCCAC TGACAACCA (SEQ ID NO: 10)
2) p53 exon 10
   Forward: GATCCGTCAT AAAGTCAAAC (SEQ ID NO: 1)
   Reverse: GGATGAGAAT GGAATCCTAT (SEQ ID No: 2)
3) ABO type gene exon 6
   Forward: CACCTGCAGA TGTGGGTGGC ACCCTGCCA (SEQ ID NO: 3)
   Reverse: GTGGAATTCA CTCGCCACTG CCTGGGTCTC (SEQ ID NO: 4)
4) ABO type gene exon 7
   Forward: GTGGCTTTCC TGAAGCTGTT C (SEQ ID NO: 5)
   Reverse: GATGCCGTTG GCCTGGTCGA C (SEQ ID No: 6)

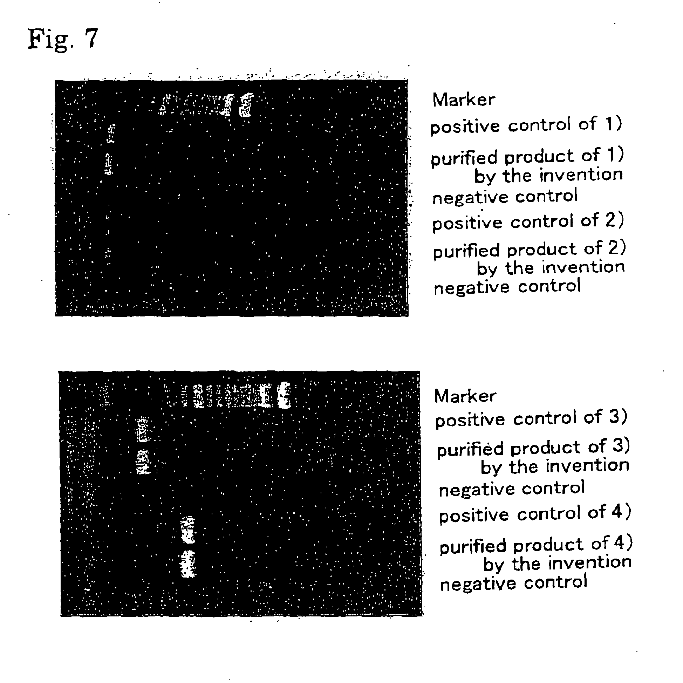

Fig. 7 shows the results of electrophoresis of the reaction products of PCR. 100 bp marker (INVITROGEN) was used. From the results of Fig. 7, it is confirmed that desired nucleic acids can be amplified by PCR using nucleic acids which were isolated and purified by the method of the present invention.

### Example 4: Detection of Pseudomonas syringae in human whole blood by nucleic acid extraction/amplification (PCR)/detection (dry analytical element for quantitative assay of pyrophosphoric acid) (model experiment of an examination of pseudomonas sepsis)

### (1) Preparation of human whole blood to which Pseudomonas syringae is added

Pseudomonas *syringae* was cultured overnight in LB medium (Luria-Bertani medium), and the culture solution was diluted with PBS so as to prepare solutions having different concentrations. These solutions were added to human whole blood which was prepared by EDTA blood collection, to prepare 6 samples of human whole blood having cell numbers of 0, 5×10⁵, 5×10⁶, 2.5×10⁶, 5×10⁷, 1×10⁸ per 1 mL respectively. The numbers of cells were assessed using a spectrophotometer.

### (2) Preparation of dry analytical element for quantitative assay of pyrophosphoric acid

An aqueous solution having a composition (a) as described in Table 4 was applied onto a smooth film sheet (a support) made of colorless transparent polyethylene terephthalate (PET) having a thickness of 180 µm, which was provided with a gelatin under-coating- The application was conducted so that after drying, a reagent layer with the following respective components was obtained.

**Table 4**

| Composition (a) of aqueous solution for reagent layer | |
|---|---|
| Gelatin | 18.8 g/m² |
| *p-*Nonylphenoxy polyxydol | 1.5 g/m² |
| (containing 10 glycidol units on average) | |
| (C₉H₁₉-Ph-O-(CH₂CH(OH)-CH₂-O)₁₀H) | |
| Xanthosine | 1.96 g/m² |
| Peroxidase | 15000 IU/m² |
| Xanthine oxidase | 13600 IU/m² |
| Purine nucleoside phosphorylase, | 3400 IU/m² |
| Leuco pigment | 0.28 g/m² |
| (2-(3,5-dimethoxy-4-hydroxyphenyl)-4-phenethyl-5-(4-dimethylaminophenyl)imidazol) | |
| Water | 136 g/m² |
| (Adjusted to have a pH of 6.8 with a dilute NaOH solution) | |

An adhesive layer aqueous solution having composition (b) as described in Table 5 was applied onto this reagent layer in such a way that after drying, an adhesive layer with the following respective components was obtained.

**Table 5**

| Composition (b) of aqueous solution for adhesive layer | |
|---|---|
| Gelatin | 3.1 g/m² |
| *p-*Nonylphenoxy polyxydol | 0.25 g/m² |
| (containing 10 glycidol units on average) | |
| (C₉H₁₉-Ph-O-(CH₂CH(OH)-CH₂-O)₁₀H) | |
| Water | 59 g/m² |

Next, a porous developing layer was provided by supplying water over the entire surface of the adhesive layer at a rate of 30 g/m² to swell the gelatin layer, followed by laminating a broad fabric of pure polyester by uniformly and slightly pressing the fabric onto the adhesive layer.

Then, an aqueous solution having composition (c) as described in Table 6 was approximately evenly applied over this developing layer in such a way that the spreading developing layer with the following respective components was obtained.
After drying and cutting into pieces having a size of 13 mm × 14 mm, the pieces were placed into a plastic mount material thereby to prepare a dry analytical element for quantitative assay of pyrophosphate.

**Table 6**

| Composition (c) of aqueous solution for developing layer | |
|---|---|
| HEPES | 2.3 g/m² |
| Sucrose | 5.0 g/m² |
| Hydroxypropylmethyl Cellulose | 0.04 g/m² |
| (containing 19% to 24% of methoxy group and 4% to 12% of hydroxypropoxy group) | |
| Pyrophosphatase | 14000 IU/ m² |
| Water | 98.6 g/m² |
| (Adjusted to have a pH of 7.2 with a dilute NaOH solution) | |

### (3) Extraction and purification of nucleic acid from human whole blood

Using 6 samples of human whole blood prepared by adding *Pseudomonas syringae* which was prepared in Process (1), nucleic acids were extracted and purified from each sample in the same manner as described in Example 2, thereby obtaining nucleic acid solutions. The nucleic acid amounts (estimated amount by a spectrophotometer) obtained from the 6 samples of human whole blood were 20 to 30 ng/µl. These obtained nucleic acid aqueous solutions were mixed aqueous solutions of human genome nucleic acids and the added Pseudomonas genome nucleic acids, and the most portions are human nucleic acids.

### (4) PCR amplification

Using nucleic acid aqueous solutions obtained by extraction and purification from the 6 samples of human whole blood in Process (3), PCR amplification was performed under the following conditions.

### <primer>

A set of primers having the following sequences specific (ice nucleation protein (InaK) N-terminal) to Pseudomonas genome nucleic acid were used.
primer (upper) ;
5'-GCGATGCTGTAATGACTCTCGACAAGC-3' (SEQ NO: 7)
primer (lower) ;
5'-GGTCTGCAAATTCTGCGGCGTCGTC-3' (SEQ ID NO: 8)

30 cycles of denaturation:94°C for 1 minute, annealing:55°C for 1 minute, and polymerase elongation reaction:72°C for 1 minute were repeated by using a reaction solution having the following composition to perform PCR amplification.

**<composition of reaction solution>**

| | |
|---|---|
| 10×PCR buffer | 5µL |
| 2.5 mM dNTP | 4µL |
| 20 µM primer (upper) | 1µL |
| 20µM primer (lower) | 1µL |
| Pyrobest | 0.25µL |
| Nucleic acid sample solution obtained in process (3) | 5µL |
| Purified water | 33.75µL |

### (5) Detection using analytical element for quantitative assay of pyrophosphoric acid

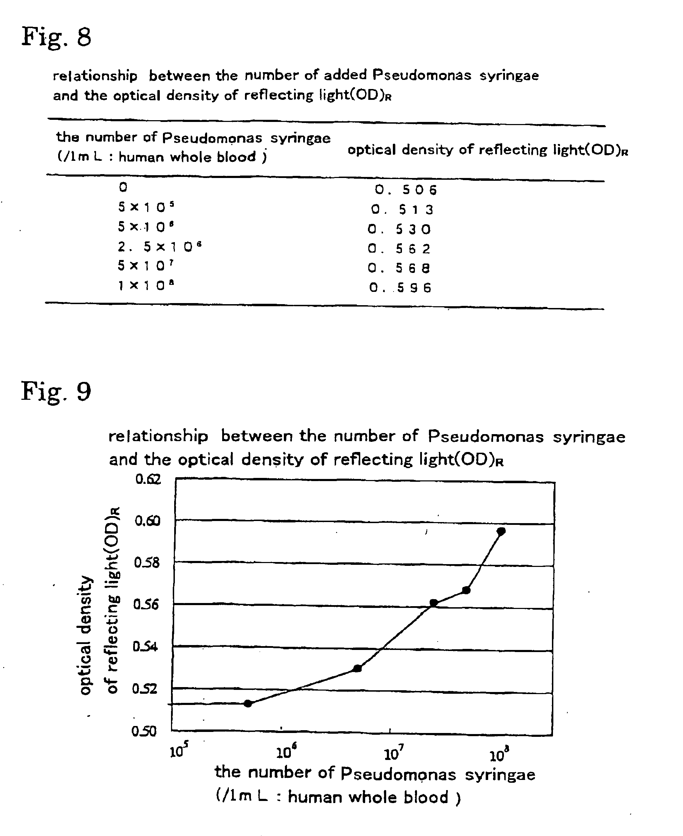

20 µl of each solution obtained after PCR amplification reaction in Process (4) was spotted onto a dry analytical element for quantitative assay of pyrophosphoric acid prepared in Process (2). Then, the dry analytical element for quantitative assay of pyrophosphoric acid was incubated for 5 minutes at 37°C, and the optical density of reflecting light (OD_{R}) was measured at a wavelength of 650 nm from the support side. The results are shown in Figs. 8 and 9.

From the result of Example 4, it is understood that the optical density of reflecting light (OD_{R}) corresponding to the amount of *Pseudomonas syringae* present in human whole blood can be obtained by performing PCR with using a nucleic acid sample solution containing a target nucleic acid fragment which was obtained from human whole blood containing *Pseudomonas syringae* by the method for isolation and purification of nucleic acid according to the present invention and using a set of primers having a sequence specific to Pseudomonas genome nucleic acid; and using the solution obtained by the PCR amplification to measure the produced pyrophosphoric acid in terms of the optical density of reflecting light (OD_{R}) with a dry analytical element for quantitative assay of pyrophosphoric acid.

### INDUSTRIAL APPLICABILITY

It is possible to isolate nucleic acids with a high purity from a sample solution containing nucleic acids by the method for isolation and purification of nucleic acid according to the present invention which uses a solid phase which has excellent isolation performance, good washing efficiency, easy workability, and can be mass produced with substantially identical isolation performance. Further, the use of the unit for isolation and purification of nucleic acid of the present invention enables easy operation.

## Claims

1. A method for isolating and purifying a nucleic acid, comprising the steps of:
(a) adsorbing a nucleic acid onto a solid phase being a porous surface-saponified cellulose triacetate membrane having a surface saponification ratio of 50% or 100%, which is present in one piece and
(b) desorbing the nucleic acid from the solid phase.

2. The method for isolating and purifying a nucleic acid according to claim 1, wherein the cellulose triacetate is coated on beads.

3. The method for isolating and purifying a nucleic acid according to one of claims 1 or 2, wherein the nucleic acid is in a sample solution.

4. The method for isolating and purifying a nucleic acid according to claim 3, wherein the sample solution is prepared by adding an aqueous organic solvent to a solution obtained by treating a sample containing a cell or a virus with a nucleic acid solubilization reagent.

5. The method for isolating and purifying a nucleic acid according to claim 4, wherein the nucleic acid solubilization reagent comprises a guanidine salt, a surfactant, and a protease.

6. The method for isolating and purifying a nucleic acid according to one of claims 1 to 5 comprising the additional step of washing the solid phase with a nucleic acid washing buffer between steps (a) and (b); and wherein step (b) is carried out by using a solution capable of desorbing the nucleic acid from the solid phase.

7. The method for isolating and purifying a nucleic acid according to claim 6, wherein the nucleic acid washing buffer contains methanol, ethanol, isopropanol or n-propanol in an amount of 20 to 100 % by weight.

8. The method for isolating and purifying a nucleic acid according to one of claims 6 or 7, wherein the solution capable of desorbing the nucleic acid from the solid phase has a salt concentration of 0.5 M or less.

9. The method for isolating and purifying a nucleic acid according to one of claims 1 to 8, wherein adsorption and desorption of the nucleic acid is performed by use of a unit for isolation and purification of nucleic acid comprising a container which has at least two openings and contains the solid phase being a porous surface-saponified cellulose triacetate membrane having a surface saponification ratio of 50% or 100%, which is present in one piece.

10. The method for isolating and purifying a nucleic acid according to one of claims 1 to 8, wherein adsorption and desorption of the nucleic acid is performed by use of a unit for isolation and purification of nucleic acid comprising:
(a) a solid phase being a porous surface-saponified cellulose triacetate membrane having a surface saponification ratio of 50% or 100%, which is present in one piece;
(b) a container having at least two openings and containing the solid phase; and
(c) a differential pressure generator connected to one opening of the container.

11. The method for isolating and purifying a nucleic acid according to claim 10, comprising the steps of:
(a) preparing a sample solution containing nucleic acids by use of a sample, and inserting one opening of the unit for isolation and purification of nucleic acid into the sample solution containing nucleic acids;
(b) creating a reduced pressure condition in a container by a differential pressure generator connected to another opening of the unit for isolation and purification of a nucleic acid, sucking the sample solution containing nucleic acids, and allowing the sample solution to come into contact with a solid phase being a porous surface-saponified cellulose triacetate membrane having a surface saponification ratio of 50% or 100%, which is present in one piece;
(c) creating an increased pressure condition in the container by the differential pressure generator connected to said another opening of the unit for isolation and purification of nucleic acid, and discharging the sucked sample solution containing nucleic acids out of the container;
(d) inserting said one opening of the unit for isolation and purification of nucleic acid into a nucleic acid washing buffer solution;
(e) creating a reduced pressure condition in the container by the differential pressure generator connected to said another opening of the unit for isolation and purification of nucleic acid, sucking the nucleic acid washing buffer solution, and allowing the buffer solution to come into contact with the solid phase;
(f) creating an increased pressure condition in the container by the differential pressure generator connected to said another opening of the unit for isolation and purification of nucleic acid, and discharging the sucked nucleic acid washing buffer solution out of the container;
(g) inserting the one opening of the unit for isolation and purification of nucleic acid into a solution capable of desorbing nucleic acids from the solid phase;
(h) creating a reduced pressure condition in the container by the differential pressure generator connected to said another opening of the unit for isolation and purification of nucleic acid, sucking the solution capable of desorbing nucleic acids from the solid phase, and allowing the solution to come into contact with the solid phase; and
(i) creating an increased pressure condition in the container by the differential pressure generator connected to said another opening of the unit for isolation and purification of nucleic acid, and discharging out of the container the solution capable of desorbing nucleic acids from the solid phase.

12. The method for isolating and purifying a nucleic acid according to claim 10, comprising the steps of:
(a) preparing a sample solution containing nucleic acids from a sample, and injecting the sample solution containing nucleic acids in one opening of a unit for isolation and purification of nucleic acid;
(b) creating an increased pressure condition in the container by a differential pressure generator connected to said one opening of the unit for isolation and purification of nucleic acid, discharging the injected sample solution containing nucleic acids out of another opening, and thereby allowing the sample solution to come into contact with a solid phase being a porous surface-saponified cellulose triacetate membrane having a surface saponification ratio of 50% or 100%, which is present in one piece;
(c) injecting a nucleic acid washing buffer in said one opening of the unit for isolation and purification of nucleic acid;
(d) creating an increased pressure condition in the container by the differential pressure generator connected to said one opening of the unit for isolation and purification of nucleic acid, discharging the injected nucleic acid washing buffer out of said another opening, and thereby allowing the buffer to come into contact with the solid phase;
(e) injecting a solution capable of desorbing nucleic acids from the solid phase in the one opening of the unit for isolation and purification of nucleic acid;
(f) creating an increased pressure condition in the container by the differential pressure generator connected to said one opening of the unit for isolation and purification of nucleic acid, discharging the injected solution capable of desorbing nucleic acids out of said another opening, and thereby desorbing nucleic acids from the solid phase and discharging the desorbed nucleic acids out of the container.

13. A unit for isolation and purification of nucleic acid comprising a container which has at least two openings and contains a solid phase being a porous surface-saponified cellulose triacetate membrane having a surface saponification ratio of 50% or 100%, which is present in one piece.

14. A unit for isolation and purification of nucleic acid according to claim 13 comprising further a differential pressure generator connected to one opening of the container.

15. The unit for isolation and purification of nucleic acid according to claim 14, wherein the differential pressure generator is detachably connected to one opening of the container.

16. The unit for isolation and purification of nucleic acid according to claim 14 or 15, wherein the differential pressure generator is an injector, a pipette or a pump.

17. The method for isolating and purifying a nucleic acid according to claim 2, wherein the beads have a cellulose triacetate membrane on a surface thereof, wherein the cellulose triacetate membrane has a hydroxyl group incorporated thereto by surface-saponification, wherein the surface saponification ratio is from 50% or 100%, which is present in one piece.

18. A method for analyzing nucleic acid comprising the steps of:
(1) isolating and purifying a nucleic acid fragment containing a target nucleic acid fragment according to the method of claim 1;
(2) allowing the target nucleic acid fragment, at least one primer complementary to a portion of the target nucleic acid fragment, at least one deoxynucleoside triphosphate, and at least one polymerase to react with each other, and performing polymerase elongation reaction with using the target nucleic acid fragment as a template and using 3' terminal of the primer as an initiation site; and
(3) detecting whether polymerase elongation reaction proceeds, or whether a polymerase elongation reaction product hybridizes with other nucleic acid.

19. The method for analyzing nucleic acid according to claim 18, wherein whether polymerase elongation reaction proceeds is detected by detecting pyrophosphoric acid which is produced in polymerase elongation reaction.

20. The method for analyzing nucleic acid according to claim 19, wherein pyrophosphoric acid is detected by a colorimetric method.

21. The method for analyzing nucleic acid according to one of claims 19 or 20, wherein pyrophosphoric acid is detected by a dry analytical element.

22. The method for analyzing nucleic acid according to claim 21, wherein the dry analytical element is a dry analytical element for quantitative assay of pyrophosphoric acid comprising a reagent layer which contains a reagent capable of converting pyrophosphoric acid into inorganic phosphorus and a group of reagents which cause color reaction depending on an amount of inorganic phosphorus.

23. The method for analyzing nucleic acid according to claim 22, wherein the dry analytical element for quantitative assay of pyrophosphoric acid comprises a reagent layer which contains xanthosine or inosine, pyrophosphatase, purine nucleoside phosphorylase, xanthine oxidase, peroxidase and a color developer.

24. The method for analyzing nucleic acid according to one of claims 18 to 23, wherein the polymerase is one selected from the group consisting of DNA polymerase I, Klenow fragment of DNA polymerase I, Bst DNA polymerase, and reverse transcriptase.

25. The method for analyzing nucleic acid according to claim 19, wherein, after pyrophosphoric acid is enzymatically converted into inorganic phosphorus, pyrophosphoric acid is detected by use of a dry analytical element for quantitative assay of inorganic phosphorus which has a reagent layer comprising xanthosine or inosine, purine nucleoside phosphorylase, xanthine oxidase, peroxidase and a color developer.

26. The method for analyzing nucleic acid according to claim 25, wherein the enzyme for converting pyrophosphoric acid into inorganic phosphorus is pyrophosphatase.

27. The method for analyzing nucleic acid according to one of claims 25 or 26, wherein the polymerase is one selected from the group consisting of DNA polymerase I, Klenow fragment of DNA polymerase I, Bst DNA polymerase, and reverse transcriptase.

28. The method for analyzing nucleic acid according to claim 18, wherein nucleic acid is assayed by the detection of the presence or abundance of the target nucleic acid fragment, or the detection of nucleotide sequence of the target nucleic acid fragment.

29. The method for analyzing nucleic acid according to claim 28, wherein the detection of nucleotide sequence of the target nucleic acid fragment is performed by detecting mutation or polymorphism of the target nucleic acid fragment.

30. An analytical apparatus for performing the method for analyzing nucleic acid of claim 18, comprising:
(1) means for isolating and purifying a nucleic acid which comprises the unit for isolation and purification of nucleic acid of claim 13;
(2) reaction means for performing polymerase elongation reaction; and
(3) means for detecting whether polymerase elongation reaction proceeds or whether a polymerase elongation reaction product hybridizes with other nucleic acid.

## Patentansprüche

1. Verfahren zur Isolierung und Aufreinigung einer Nucleinsäure, umfassend die Schritte:
(a) Adsorbieren einer Nucleinsäure an einer festen Phase, die eine poröse oberflächenverseifte Cellulosetriacetat-Membran ist, die ein Oberflächenverseifungsverhältnis von 50% oder 100% hat, die in einem Stück vorliegt, und
(b) Desorbieren der Nucleinsäure von der festen Phase.

2. Verfahren zur Isolierung und Aufreinigung einer Nucleinsäure gemäß Anspruch 1, wobei das Cellulosetriacetat auf Perlen aufgetragen ist.

3. Verfahren zur Isolierung und Aufreinigung einer Nucleinsäure gemäß einem der Ansprüche 1 oder 2, wobei die Nucleinsäure in einer Probenlösung ist.

4. Verfahren zur Isolierung und Aufreinigung einer Nucleinsäure gemäß Anspruch 3, wobei die Probenlösung hergestellt wird, indem ein wässriges organisches Lösungsmittel zu einer Lösung gegeben wird, welche erhalten wird, indem eine Probe, die eine Zelle oder ein Virus enthält, mit einem Nucleinsäure-Solubilisierungs-Reagens behandelt wird.

5. Verfahren zur Isolierung und Aufreinigung einer Nucleinsäure gemäß Anspruch 4, wobei das Nucleinsäure-Solubilisierungs-Reagens ein Guanidinsalz, ein oberflächenaktives Mittel und eine Protease umfasst.

6. Verfahren zur Isolierung und Aufreinigung einer Nucleinsäure gemäß einem der Ansprüche 1 bis 5, das den zusätzlichen Schritt eines Waschens der festen Phase mit einem Nucleinsäure-Waschpuffer zwischen den Schritte (a) und (b) umfasst, und wobei Schritt (b) durchgeführt wird, indem eine Lösung verwendet wird, die fähig ist, die Nucleinsäure von der festen Phase zu desorbieren.

7. Verfahren zur Isolierung und Aufreinigung einer Nucleinsäure gemäß Anspruch 6, wobei der Nucleinsäure-Waschpuffer Methanol, Ethanol, Isopropanol oder n-Propanol in einer Menge von 20 bis 100 Gew.-% enthält.

8. Verfahren zur Isolierung und Aufreinigung einer Nucleinsäure gemäß einem der Ansprüche 6 oder 7, wobei die Lösung, die fähig ist, die Nucleinsäure von der festen Phase zu desorbieren, eine Salzkonzentration von 0,5 M oder weniger hat.

9. Verfahren zur Isolierung und Aufreinigung einer Nucleinsäure gemäß einem der Ansprüche 1 bis 8, wobei die Adsorption und die Desorption der Nucleinsäure durch Verwendung einer Einheit zur Isolierung und Aufreinigung einer Nucleinsäure durchgeführt werden, welche einen Behälter umfasst, welcher wenigstens zwei Öffnungen hat und die feste Phase, die eine poröse oberflächenverseifte Cellulosetriacetat-Membran ist, die ein Oberflächenverseifungsverhältnis von 50% oder 100% hat, die in einem Stück vorliegt, enthält.

10. Verfahren zur Isolierung und Aufreinigung einer Nucleinsäure gemäß einem der Ansprüche 1 bis 8, wobei die Adsorption und die Desorption der Nucleinsäure durchgeführt werden durch Verwendung einer Einheit zur Isolierung und Aufreinigung einer Nucleinsäure, umfassend:
(a) eine feste Phase, die eine poröse oberflächenverseifte Cellulosetriacetat-Membran ist, die ein Oberflächenverseifungsverhältnis von 50% oder 100% hat, die in einem Stück vorliegt;
(b) einen Behälter, der wenigstens zwei Öffnungen hat und die feste Phase enthält, und
(c) einen Differenzdruckgenerator, der an eine Öffnung des Behälters angeschlossen ist.

11. Verfahren zur Isolierung und Aufreinigung einer Nucleinsäure gemäß Anspruch 10, umfassend die Schritte:
(a) Herstellen einer Probenlösung, die Nucleinsäuren enthält, durch Verwendung einer Probe und Einsetzen einer Öffnung der Einheit zur Isolierung und Aufreinigung von Nucleinsäure in die Probenlösung, die Nucleinsäuren enthält;
(b) Erzeugen eines reduzierten Druckzustandes in einem Behälter durch einen Differenzdruckgenerator, der an eine andere Öffnung der Einheit zur Isolierung und Aufreinigung einer Nucleinsäure angeschlossen ist, Ansaugen der Probenlösung, die Nucleinsäuren enthält, und Inkontaktkommenlassen der Probenlösung mit einer festen Phase, die eine poröse oberflächenverseifte Cellulosetriacetat-Membran ist, die ein Oberflächenverseifungsverhältnis von 50% oder 100% hat, die in einem Stück vorliegt;
(c) Erzeugen eines erhöhten Druckzustandes im Behälter durch den Differenzdruckgenerator, der an die andere Öffnung der Einheit zur Isolierung und Aufreinigung von Nucleinsäure angeschlossen ist, und Ausbringen der angesaugten Probenlösung, die Nucleinsäuren enthält, aus dem Behälter;
(d) Einsetzen der einen Öffnung der Einheit zur Isolierung und Aufreinigung von Nucleinsäure in eine Nucleinsäure-Waschpufferlösung;
(e) Erzeugen eines reduzierten Druckzustandes in dem Behälter durch den Differenzdruckgenerator, der an die andere Öffnung der Einheit zur Isolierung und Aufreinigung von Nucleinsäure angeschlossen ist, Ansaugen der Nucleinsäure-Waschpufferlösung und Inkontaktkommenlassen der Pufferlösung mit der festen Phase;
(f) Erzeugen eines erhöhten Druckzustandes in dem Behälter durch den Differenzdruckgenerator, der an die andere Öffnung der Einheit zur Isolierung und Aufreinigung von Nucleinsäure angeschlossen ist, und Ausbringen der angesaugten Nucleinsäure-Waschpufferlösung aus dem Behälter;
(g) Einsetzen der einen Öffnung der Einheit zur Isolierung und Aufreinigung von Nucleinsäure in eine Lösung, die fähig ist, Nucleinsäuren von der festen Phase zu desorbieren;
(h) Erzeugen eines reduzierten Druckzustandes in dem Behälter durch den Differenzdruckgenerator, der an die andere Öffnung der Einheit zur Isolierung und Aufreinigung von Nucleinsäure angeschlossen ist, Ansaugen der Lösung, die fähig ist, Nucleinsäuren von der festen Phase zu desorbieren, und Inkontaktkommenlassen der Lösung mit der festen Phase und
(i) Erzeugen eines erhöhten Druckzustandes in dem Behälter durch den Differenzdruckgenerator, der an die andere Öffnung der Einheit zur Isolierung und Aufreinigung von Nucleinsäure angeschlossen ist, und Ausbringen der Lösung, die fähig ist, Nucleinsäuren von der festen Phase zu desorbieren, aus dem Behälter.

12. Verfahren zur Isolierung und Aufreinigung einer Nucleinsäure gemäß Anspruch 10, umfassend die Schritte:
(a) Herstellen einer Probenlösung, die Nucleinsäuren enthält, aus einer Probe und Injizieren der Probenlösung, die Nucleinsäuren enthält, in eine Öffnung einer Einheit zur Isolierung und Aufreinigung von Nucleinsäure;
(b) Erzeugen eines erhöhten Druckzustandes in dem Behälter durch einen Differenzdruckgenerator, der an eine Öffnung der Einheit zur Isolierung und Aufreinigung von Nucleinsäure angeschlossen ist, Ausbringen der injizierten Probenlösung, die Nucleinsäuren enthält, aus einer anderen Öffnung und **dadurch** Inkontaktkommenlassen der Probenlösung mit einer festen Phase, die eine poröse oberflächenverseifte Cellulosetriacetat-Membran ist, die ein Oberflächenverseifungsverhältnis von 50% oder 100% hat, die in einem Stück vorliegt;
(c) Injizieren eines Nucleinsäure-Waschpuffers in eine Öffnung der Einheit zur Isolierung und Reinigung von Nucleinsäure;
(d) Erzeugen eines erhöhten Druckzustandes in dem Behälter durch den Differenzdruckgenerator, der an eine Öffnung der Einheit zur Isolierung und Reinigung von Nucleinsäure angeschlossen ist, Ausbringen des injizierten Nucleinsäure-Waschpuffers aus der anderen Öffnung und **dadurch** Inkontaktkommenlassen des Puffers mit der festen Phase;
(e) Injizieren einer Lösung, die fähig ist, Nucleinsäuren von der festen Phase zu desorbieren, in eine Öffnung der Einheit zur Isolierung und Aufreinigung von Nucleinsäure;
(f) Erzeugen eines erhöhten Druckzustandes in dem Behälter durch den Differenzdruckgenerator, der an eine Öffnung der Einheit zur Isolierung und Aufreinigung von Nucleinsäure angeschlossen ist, Ausbringen der injizierten Lösung, die fähig ist, Nucleinsäuren zu desorbieren, aus der anderen Öffnung und **dadurch** Desorbieren von Nucleinsäuren von der festen Phase, und Ausbringen der desorbierten Nucleinsäuren aus dem Behälter.

13. Einheit zur Isolierung und Aufreinigung von Nucleinsäure, umfassend einen Behälter, der wenigstens zwei Öffnungen hat und eine feste Phase enthält, die eine poröse oberflächenverseifte Cellulosetriacetat-Membran ist, die ein Oberflächenverseifungsverhältnis von 50% oder 100% hat, die in einem Stück vorliegt.

14. Einheit zur Isolierung und Aufreinigung von Nucleinsäure gemäß Anspruch 13, die außerdem einen Differenzdruckgenerator umfasst, der an eine Öffnung des Behälters angeschlossen ist.

15. Einheit zur Isolierung und Aufreinigung von Nucleinsäure gemäß Anspruch 14, wobei der Differenzdruckgenerator abnehmbar an eine Öffnung des Behälters angeschlossen ist.

16. Einheit zur Isolierung und Aufreinigung von Nucleinsäure gemäß Anspruch 14 oder 15, wobei der Differenzdruckgenerator ein Injektor, eine Pipette oder eine Pumpe ist.

17. Verfahren zur Isolierung und Aufreinigung einer Nucleinsäure gemäß Anspruch 2, wobei die Perlen eine Cellulosetriacetat-Membran an einer Oberfläche haben, wobei die Cellulosetriacetat-Membran eine Hydroxylgruppe durch Oberflächenverseifung eingebaut hat, wobei das Oberflächenverseifungsverhältnis 50 oder 100% ist, wobei diese in einem Stück vorliegt.

18. Verfahren zum Analysieren von Nucleinsäure, umfassend die Schritte:
(1) Isolieren und Reinigen eines Nucleinsäurefragments, das ein Targetnucleinsäurefragment enthält, gemäß dem Verfahren von Anspruch 1;
(2) Reagieren lassen des Targetnucleinsäurefragments wenigstens eines Primers, der komplementär zu einem Teil des Targetnucleinsäurefragments ist, wenigstens eines Desoxynucleosidtriphosphats und wenigstens einer Polymerase miteinander und Durchführen einer Polymeraseverlängerungsreaktion unter Verwendung des Targetnucleinsäurefragments als Matrize und unter Verwendung des 3'-Terminus des Primers als Initiierungsstelle und
(3) Detektieren, ob eine Polymeraseverlängerungsreaktion fortschreitet oder ob ein Polymeraseverlängerungsreaktionsprodukt mit anderer Nucleinsäure hybridisiert.

19. Verfahren zum Analysieren von Nucleinsäure gemäß Anspruch 18, wobei detektiert wird, ob eine Polymeraseverlängerungsreaktion fortschreitet, indem Pyrophosphorsäure detektiert wird, welche in einer Polymeraseverlängerungsreaktion produziert wird.

20. Verfahren zum Analysieren von Nucleinsäure gemäß Anspruch 19, wobei Pyrophosphorsäure durch ein kolorimetrisches Verfahren detektiert wird.

21. Verfahren zum Analysieren von Nucleinsäure gemäß einem der Ansprüche 19 oder 20, wobei Pyrophosphorsäure durch ein Trockenanalyseelement detektiert wird.

22. Verfahren zum Analysieren von Nucleinsäure gemäß Anspruch 21, wobei das Trockenanalyseelement ein Trockenanalyseelement für einen quantitativen Pyrophosphorsäure-Assay ist, umfassend eine Reagensschicht, welche eine Reagens, das fähig ist, Pyrophosphorsäure in anorganischen Phosphor umzuwandeln, und eine Gruppe von Reagenzien, welche in Abhängigkeit von einer Menge an anorganischem Phosphor eine Farbreaktion bewirken, enthält.

23. Verfahren zum Analysieren von Nucleinsäure gemäß Anspruch 22, wobei das Trockenanalyseelement für einen quantitativen Pyrophosphorsäure-Assay eine Reagensschicht umfasst, welche Xanthosin oder Inosin, Pyrophosphatase, Purinnucleosidphosphorylase, Xanthinoxidase, Peroxidase und einen Farbentwickler enthält.

24. Verfahren zum Analysieren von Nucleinsäure gemäß einem der Ansprüche 18 bis 23, wobei die Polymerase eine ist, die ausgewählt ist aus der Gruppe, bestehend aus DNA-Polymerase I, Klenow-Fragment von DNA-Polymerase I, Bst-DNA-Polymerase und reverser Transkriptase.

25. Verfahren zum Analysieren von Nucleinsäure gemäß Anspruch 19, wobei, nachdem Pyrophosphorsäure enzymatisch in anorganischen Phosphor umgewandelt wurde, Pyrophosphorsäure durch Verwendung eines Trockenanalyseelements für einen quantitativen Assay von anorganischem Phosphor detektiert wird, wobei dieses eine Reagensschicht hat, die Xanthosin oder Inosin, Purinnucleosidphosphorylase, Xanthinoxidase, Peroxidase und einen Farbentwickler umfasst.

26. Verfahren zum Analysieren von Nucleinsäure gemäß Anspruch 25, wobei das Enzym zum Umwandeln von Pyrophosphorsäure in anorganischen Phosphor Pyrophosphatase ist.

27. Verfahren zum Analysieren von Nucleinsäure gemäß einem der Ansprüche 25 oder 26, wobei die Polymerase eine ist, die ausgewählt wird aus der Gruppe, bestehend aus DNA-Polymerase I, Klenow-Fragment von DNA-Polymerase I, Bst-DNA-Polymerase und reverser Transkriptase.

28. Verfahren zum Analysieren von Nucleinsäure gemäß Anspruch 18, wobei Nucleinsäure durch die Detektion des Vorliegens oder der Abundanz des Targetnucleinsäurefragments oder die Detektion einer Nucleotidsequenz des Targetnucleinsäurefragments analysiert wird.

29. Verfahren zum Analysieren von Nucleinsäure gemäß Anspruch 28, wobei die Detektion einer Nucleotidsequenz des Targetnucleinsäurefragments durchgeführt wird, indem Mutation oder Polymorphismus des Targetnucleinsäurefragments detektiert wird.

30. Analysegerät zur Durchführung des Verfahrens zum Analysieren von Nucleinsäure gemäß Anspruch 18, umfassend:
(1) Mittel zur Isolierung und Aufreinigung einer Nucleinsäure, das die Einheit zur Isolierung und Aufreinigung von Nucleinsäure gemäß Anspruch 13 umfasst;
(2) Reaktionsmittel zur Durchführung einer Polymeraseverlängerungsreaktion und
(3) Mittel zum Detektieren, ob eine Polymeraseverlängerungsreaktion fortschreitet oder ob ein Polymeraseverlängerungsreaktionsprodukt mit anderer Nucleinsäure hybridisiert.

## Revendications

1. Procédé pour isoler et purifier un acide nucléique, comprenant les étapes de :
(a) adsorber un acide nucléique sur une phase solide qui est une membrane poreuse de triacétate de cellulose saponifiée en surface ayant un taux de saponification de la surface de 50 % ou 100 %, qui est présente en une pièce et
(b) désorber l'acide nucléique de la phase solide.

2. Procédé pour isoler et purifier un acide nucléique selon la revendication 1 où le triacétate de cellulose est appliqué en revêtement sur des billes.

3. Procédé pour isoler et purifier un acide nucléique selon l'une des revendications 1 et 2 où l'acide nucléique est dans une solution d'échantillon.

4. Procédé pour isoler et purifier un acide nucléique selon la revendication 3 où la solution d'échantillon est préparée par addition d'un solvant organique aqueux à une solution obtenue par traitement d'un échantillon contenant une cellule ou un virus avec un réactif de solubilisation d'acide nucléique.

5. Procédé pour isoler et purifier un acide nucléique selon la revendication 4 où le réactif de solubilisation d'acide nucléique comprend un sel de guanidine, un tensioactif et une protéase.

6. Procédé pour isoler et purifier un acide nucléique selon l'une des revendications 1 à 5 comprenant l'étape supplémentaire de lavage de la phase solide avec un tampon de lavage d'acide nucléique entre les étapes (a) et (b) ; et où l'étape (b) est réalisée en utilisant une solution capable de désorber l'acide nucléique de la phase solide.

7. Procédé pour isoler et purifier un acide nucléique selon la revendication 6 où le tampon de lavage d'acide nucléique contient du méthanol, de l'éthanol, de l'isopropanol ou du n-propanol en une quantité de 20 à 100 % en poids.

8. Procédé pour isoler et purifier un acide nucléique selon l'une des revendications 6 et 7 où la solution capable de désorber l'acide nucléique de la phase solide a une concentration de sels de 0,5 M ou moins.

9. Procédé pour isoler et purifier un acide nucléique selon l'une des revendications 1 à 8 où l'adsorption et la désorption de l'acide nucléique sont accomplies au moyen d'une unité pour isoler et purifier un acide nucléique comprenant un récipient qui a au moins deux ouvertures et qui contient la phase solide qui est une membrane poreuse de triacétate de cellulose saponifiée en surface ayant un taux de saponification de la surface de 50 % ou 100 %, qui est présente en une pièce.

10. Procédé pour isoler et purifier un acide nucléique selon l'une des revendications 1 à 8 où l'adsorption et la désorption de l'acide nucléique sont réalisées au moyen d'une unité pour isoler et purifier un acide nucléique comprenant :
(a) une phase solide qui est une membrane poreuse de triacétate de cellulose saponifiée en surface et ayant un taux de saponification de la surface de 50 % ou 100 %, qui est présente en une pièce ;
(b) un récipient ayant au moins deux ouvertures et contenant la phase solide ; et
(c) un générateur de pression différentielle relié à une ouverture du récipient.

11. Procédé pour isoler et purifier un acide nucléique selon la revendication 10 comprenant les étapes de :
(a) préparer une solution d'échantillon contenant des acides nucléiques au moyen d'un échantillon, et insérer une ouverture de l'unité pour isoler et purifier un acide nucléique dans la solution d'échantillon contenant des acides nucléiques ;
(b) créer des conditions de pression réduite dans un récipient par un générateur de pression différentielle relié à une autre ouverture de l'unité pour isoler et purifier un acide nucléique, aspirer la solution d'échantillon contenant des acides nucléiques et permettre à la solution d'échantillon de venir en contact avec une phase solide qui est une membrane poreuse de triacétate de cellulose saponifiée en surface ayant un taux de saponification de la surface de 50 % ou 100 %, qui est présente en une pièce ;
(c) créer des conditions de pression accrue dans le récipient par le générateur de pression différentielle relié à ladite autre ouverture de l'unité pour isoler et purifier un acide nucléique, et évacuer du récipient la solution d'échantillon contenant des acides nucléiques aspirée ;
(d) insérer ladite une ouverture de l'unité pour isoler et purifier un acide nucléique dans une solution de tampon de lavage d'acide nucléique ;
(e) créer des conditions de pression réduite dans le récipient par le générateur de pression différentielle relié à ladite autre ouverture de l'unité pour isoler et purifier un acide nucléique, aspirer la solution de tampon de lavage d'acide nucléique et permettre à la solution de tampon de venir en contact avec la phase solide ;
(f) créer des conditions de pression accrue dans le récipient par le générateur de pression différentielle relié à ladite autre ouverture de l'unité pour isoler et purifier un acide nucléique, et évacuer du récipient la solution de tampon de lavage d'acide nucléique aspirée ;
(g) insérer la une ouverture de l'unité pour isoler et purifier un acide nucléique dans une solution capable de désorber des acides nucléiques de la phase solide ;
(h) créer des conditions de pression réduite dans le récipient par le générateur de pression différentielle relié à ladite autre ouverture de l'unité pour isoler et purifier un acide nucléique, aspirer la solution capable de désorber des acides nucléiques de la phase solide, et permettre à la solution de venir en contact avec la phase solide ; et
(i) créer des conditions de pression accrue dans le récipient par le générateur de pression différentielle relié à ladite autre ouverture de l'unité pour isoler et purifier un acide nucléique, et évacuer du récipient la solution capable de désorber des acides nucléiques de la phase solide.

12. Procédé pour isoler et purifier un acide nucléique selon la revendication 10 comprenant les étapes de :
(a) préparer une solution d'échantillon contenant des acides nucléiques à partir d'un échantillon et injecter la solution d'échantillon contenant des acides nucléiques dans une ouverture d'une unité pour isoler et purifier un acide nucléique ;
(b) créer des conditions de pression accrue dans le récipient par un générateur de pression différentielle relié à ladite une ouverture de l'unité pour isoler et purifier un acide nucléique, évacuer de l'autre ouverture la solution d'échantillon contenant des acides nucléiques injectée, et permettre ainsi à la solution d'échantillon de venir en contact avec une phase solide qui est une membrane poreuse de triacétate de cellulose saponifiée en surface ayant un taux de saponification de la surface de 50 % ou 100 %, qui est présente en une pièce ;
(c) injecter un tampon de lavage d'acide nucléique dans ladite une ouverture de l'unité pour isoler et purifier un acide nucléique ;
(d) créer des conditions de pression accrue dans le récipient par le générateur de pression différentielle relié à ladite une ouverture de l'unité pour isoler et purifier un acide nucléique, évacuer de ladite autre ouverture le tampon de lavage d'acide nucléique injecté et permettre ainsi au tampon de venir en contact avec la phase solide ;
(e) injecter une solution capable de désorber des acides nucléiques de la phase solide dans la une ouverture de l'unité pour isoler et purifier un acide nucléique ;
(f) créer des conditions de pression accrue dans le récipient par le générateur de pression différentielle relié à ladite une ouverture de l'unité pour isoler et purifier un acide nucléique, évacuer de ladite autre ouverture la solution capable de désorber des acides nucléiques injectée, et désorber ainsi les acides nucléiques de la phase solide et évacuer du récipient les acides nucléiques désorbés.

13. Unité pour isoler et purifier un acide nucléique comprenant un récipient qui a au moins deux ouvertures et qui contient une phase solide qui est une membrane poreuse de triacétate de cellulose saponifiée en surface ayant un taux de saponification de la surface de 50 % ou 100 %, qui est présente en une pièce.

14. Unité pour isoler et purifier un acide nucléique selon la revendication 13 comprenant en outre un générateur de pression différentielle relié à une ouverture du récipient.

15. Unité pour isoler et purifier un acide nucléique selon la revendication 14 où le générateur de pression différentielle est relié de manière amovible à une ouverture du récipient.

16. Unité pour isoler et purifier un acide nucléique selon la revendication 14 ou 15 où le générateur de pression différentielle est un injecteur, une pipette ou une pompe.

17. Procédé pour isoler et purifier un acide nucléique selon la revendication 2 où les billes ont une membrane de triacétate de cellulose sur une surface de celles-ci, où la membrane de triacétate de cellulose a un groupe hydroxyle incorporé dans celle-ci par saponification de la surface, où le taux de saponification de la surface est de 50 % ou 100 %, qui est présente en une pièce.

18. Procédé pour analyser un acide nucléique comprenant les étapes de :
(1) isoler et purifier un fragment d'acide nucléique contenant un fragment d'acide nucléique cible selon le procédé de la revendication 1 ;
(2) permettre au fragment d'acide nucléique cible, à au moins une amorce complémentaire d'une partie du fragment d'acide nucléique cible, à au moins un désoxynucléoside triphosphate et à au moins une polymérase de réagir entre eux, et conduire une réaction d'élongation par polymérase en utilisant le fragment d'acide nucléique cible comme matrice et en utilisant l'extrémité 3' de l'amorce comme site d'initiation ; et
(3) détecter si une réaction d'élongation par polymérase se déroule, ou si un produit de réaction d'élongation par polymérase s'hybride avec un autre acide nucléique.

19. Procédé pour analyser un acide nucléique selon la revendication 18 où le fait qu'une réaction d'élongation par polymérase se déroule est détecté par détection de l'acide pyrophosphorique qui est produit dans la réaction d'élongation par polymérase.

20. Procédé pour analyser un acide nucléique selon la revendication 19 où l'acide pyrophosphorique est détecté par un procédé colorimétrique.

21. Procédé pour analyser un acide nucléique selon l'une des revendications 19 et 20 où l'acide pyrophosphorique est détecté par un élément analytique sec.

22. Procédé pour analyser un acide nucléique selon la revendication 21 où l'élément analytique sec est un élément analytique sec pour un test quantitatif de l'acide pyrophosphorique comprenant une couche de réactifs qui contient un réactif capable de convertir l'acide pyrophosphorique en phosphore inorganique et un groupe de réactifs qui entraînent une réaction colorée selon la quantité de phosphore inorganique.

23. Procédé pour analyser un acide nucléique selon la revendication 22 où l'élément analytique sec pour le test quantitatif de l'acide pyrophosphorique comprend une couche de réactifs qui contient de la xanthosine ou de l'inosine, de la pyrophosphatase, de la purine nucléoside phosphorylase, de la xanthine oxydase, de la peroxydase et un développateur chromogène.

24. Procédé pour analyser un acide nucléique selon l'une des revendications 18 à 23 où la polymérase est une polymérase choisie dans le groupe consistant en l'ADN polymérase I, le fragment de Klenow de l'ADN polymérase I, l'ADN polymérase Bst et la transcriptase inverse.

25. Procédé pour analyser un acide nucléique selon la revendication 19 où, après la conversion enzymatique de l'acide pyrophosphorique en phosphore inorganique, l'acide pyrophosphorique est détecté au moyen d'un élément analytique sec pour le test quantitatif du phosphore inorganique qui a une couche de réactifs comprenant de la xanthosine ou de l'inosine, de la purine nucléoside phosphorylase, de la xanthine oxydase, de la peroxydase et un développateur chromogène.

26. Procédé pour analyser un acide nucléique selon la revendication 25 où l'enzyme pour convertir l'acide pyrophosphique en phosphore inorganique est la pyrophosphatase.

27. Procédé pour analyser un acide nucléique selon l'une des revendications 25 et 26 où la polymérase et une polymérase choisie dans le groupe consistant en l'ADN polymérase I, le fragment de Klenow de l'ADN polymérase I, l'ADN polymérase Bst et la transcriptase inverse.

28. Procédé pour analyser un acide nucléique selon la revendication 18 où l'acide nucléique est testé par la détection de la présence ou de l'abondance du fragment d'acide nucléique cible, ou la détection de la séquence nucléotidique du fragment d'acide nucléique cible.

29. Procédé pour analyser un acide nucléique selon la revendication 28 où la détection de la séquence nucléotidique du fragment d'acide nucléique cible est réalisée par détection d'une mutation ou d'un polymorphisme du fragment d'acide nucléique cible.

30. Appareil analytique pour mettre en oeuvre le procédé pour analyser un acide nucléique selon la revendication 18, comprenant :
(1) des moyens pour isoler et purifier un acide nucléique qui comprennent l'unité pour isoler et purifier un acide nucléique selon la revendication 13 ;
(2) des moyens réactionnels pour conduire une réaction d'élongation par polymérase ; et
(3) des moyens pour détecter si une réaction d'élongation par polymérase se déroule ou si un produit de réaction d'élongation par polymérase s'hybride avec un autre acide nucléique.
